(19) Europäisches Patentamt
European Patent Office
Office européen des brevets

(11) **EP 4 134 675 A1**

(12) **EUROPEAN PATENT APPLICATION**
published in accordance with Art. 153(4) EPC

(43) Date of publication:
**15.02.2023 Bulletin 2023/07**

(21) Application number: **21785245.8**

(22) Date of filing: **07.04.2021**

(51) International Patent Classification (IPC):
**G01N 33/86** (1980.01)

(52) Cooperative Patent Classification (CPC):
**G01N 33/86**

(86) International application number:
**PCT/JP2021/014697**

(87) International publication number:
**WO 2021/206107 (14.10.2021 Gazette 2021/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.04.2020 JP 2020068877**

(71) Applicant: **Sekisui Medical Co., Ltd.
Tokyo 103-0027 (JP)**

(72) Inventor: **KAWABE, Toshiki
Tokyo 103-0027 (JP)**

(74) Representative: **Hartz, Nikolai
Wächtershäuser & Hartz
Patentanwaltspartnerschaft mbB
Weinstrasse 8
80333 München (DE)**

(54) **METHOD FOR MEASURING BLOOD COAGULATION TIME**

(57) Provided is a method for accurately measuring coagulation time of blood specimens showing various coagulation reactions. The method for measuring blood coagulation time includes acquiring coagulation reaction P(i) of a subject specimen and reaction velocity V(i); calculating a calculation start point Te by using the ratio of the V(i) to maximum reaction velocity as an index; and calculating time Tc giving P(Tc) = P(Te) × N% (0 < N < 100) and determining the Tc as the blood coagulation time.

EP 4 134 675 A1

**Description**

Field of the Invention

The present invention relates to a method for measuring blood coagulation time.

Background of the Invention

A blood coagulation test is a test for diagnosing blood coagulability of a patient by adding a prescribed reagent to a blood specimen of the patient and measuring, for example, the blood coagulation time. Typical examples of the blood coagulation time include prothrombin time (PT), activated partial thromboplastin time (APTT), and thrombin time. The blood coagulation test can examine the hemostatic ability and fibrinolytic capacity of a patient. An abnormality in the blood coagulation ability mainly causes elongation of the coagulation time. For example, elongation of coagulation time is caused by, for example, effect of an anticoagulant, reduction of a coagulation-participating component, congenital deficiency of a blood coagulation factor, and an acquired autoantibody that inhibits coagulation reaction.

In recent years, automated analyzers that perform automatic measurement for blood coagulation tests are being widely used, and blood coagulation tests can be easily carried out. For example, in some kind of automated analyzer, a mixed solution obtained by adding a reagent to a blood specimen is illuminated with light, and the coagulation reaction of the blood specimen is measured based on the resulting change in the amount of light. For example, when the amount of scattered light is measured, the scattered light intensity is sharply increased, by progression of coagulation, at a point of time after a certain amount of time has passed since the addition of the reagent to a blood specimen, then saturates as the coagulation reaction approaches the end, and reaches the plateau. The blood coagulation time can be measured based on such a temporal change in scattered light intensity.

As the method for calculating coagulation time by an automated analyzer, several procedures, such as a percentage method and a differentiation method, are used (see Patent Literature 1). When the coagulation time is calculated based on the amount of scattered light, in a percentage method, typically, the time until the measured scattered light intensity reaches a certain percentage of the maximum value thereof is calculated as the coagulation time. The percentage method can quite accurately calculate the coagulation time not only of a normal sample but also of an abnormal sample, such as a low-fibrinogen sample, a chyle sample, and a hemolyzed blood sample. However, the automatic analysis based on the percentage method needs to set the sample measurement time long such that the maximum amount of scattered light can be detected even in an abnormal sample with low coagulability, such as a low-fibrinogen sample. Therefore, the analysis takes time.

In a differentiation method, typically, the time until the differential value of the amount of scattered light reaches the peak or a certain percentage thereof is calculated as the coagulation time. However, in the abnormal sample with low coagulability, such as a low-fibrinogen sample, no clear peak of the differential value of the amount of scattered light is observed in some cases. In addition, in the abnormal sample, two or more peaks of the differential value are observed in some cases. A procedure of calculating the coagulation time based on a single-peak curve formed by fitting of a differential value curve may be used, the fitting impairs the accurate information relating to the coagulability of a sample in some cases.

Furthermore, photometric data from an analyzer include various noises caused by the apparatus, the reagent, and the condition of the sample, and the noises may produce false detection of the coagulation time. The automatic analysis of a blood specimen is required to calculate reliable coagulation time by removing the bad influence of noises.

Citation List

Patent Literature

Patent Literature 1: JP-A-6-249855

Summary of the Invention

Technical Problem

The present invention relates to a method for measuring blood coagulation time that can accurately measure coagulation time of blood specimens showing various blood coagulation reaction curves.

Solution to Problem

That is, the present invention provides the followings:

[1] A method for measuring blood coagulation time, comprising:

acquiring coagulation reaction P(i) of a subject specimen and reaction velocity V(i) as a differential value of the reaction P(i), where i represents a measuring point;

calculating a calculation start point Te by using a ratio of V(i) to maximum reaction velocity as an index; and

calculating time Tc giving $P(Tc) = P(Te) \times N\%$ (0 < N < 100) and determining the Tc as blood coagulation time;

[2] The method according to [1], wherein the maximum reaction velocity is a maximum value of V(i) acquired so far;
[3] The method according to [1] or [2], wherein the maximum reaction velocity is a maximum value of V(i) acquired after V(i) exceeds a threshold Vs until a latest measuring point;
[4] The method according to any one of [1] to [3], wherein time at which V(i) reaches S% (S is a prescribed value within a range of from 5 to 95) of a maximum reaction velocity after V(i) arrives at the maximum reaction velocity is calculated as the calculation start point Te;
[5] The method according to [4], wherein

time at which V(i) reaches S% (S is a prescribed value within a range of from 5 to 95) of a maximum reaction velocity in a case where a maximum value continuation width K(i) exceeds a threshold Ks after V(i) arrived at the maximum reaction velocity is calculated as the calculation start point Te, where
the maximum value continuation width K(i) is the time or the number of measuring points at which V(i) at i continues to be a certain value after arriving at the maximum reaction velocity;

[6] The method according to [4] or [5], wherein

time at which V(i) is S% (S is a prescribed value within a range of from 5 to 95) of the maximum reaction velocity in a case where a peak width W(i) exceeds a threshold Ws is calculated as the calculation start point Te, where
the peak width W(i) is a time width or number of measuring points between a latest i and a point at which V(i) is X% of the maximum reaction velocity before arriving at the maximum reaction velocity, where
X is 1 or more and less than 100;

[7] A method for measuring coagulation factor concentration, comprising measuring coagulation factor concentration of a subject specimen based on blood coagulation time of the subject specimen measured by the method according to any one of the [1] to [6]; and
[8] The method according to [7], wherein the coagulation factor is fibrinogen.

Advantageous Effects of the Invention

According to the method of the present invention, coagulation times of blood specimens showing various blood coagulation reaction curves including a normal sample and an abnormal sample can be accurately measured. In addition, when a large number of samples are analyzed at real time with an automated analyzer, the method of the present invention can shorten the analysis time for one sample and can improve the analysis efficiency, compared to a conventional percentage method.

Brief Description of Drawings

[Figure 1] Figure 1 is an example of a coagulation reaction curve.
[Figure 2] Figure 2 is a basic flow chart of an embodiment of a method for measuring blood coagulation time of the present invention.
[Figure 3] Figure 3 shows reaction P(i) and reaction velocity V(i). The left is a normal sample, and the right is a FVIII deficiency sample.
[Figure 4] Figure 4 shows a conceptual scheme of a procedure for determining Vmax. S1 to S6 show, in the time series, V(i) acquired during from a point of time (T1) of the reaction initial stage to a point of time (Tt) of Vmax determination.

[Figure 5] Figure 5 shows relationships between V(i) and Vp(i), W(i), or K(i). A to D: normal samples, and E to H: FVIII deficiency samples.

[Figure 6] Figure 6 shows examples of measured Te and Tc, and APTT by a percentage method. A: APTT minimum sample, and B: APTT maximum sample. In the graphs, P(i) (the above) and V(i) (the below) are shown together with the point of time of Pmax (rhombus), APTT (triangle) by a percentage method based on Pmax, $Te_{40}$ and $Te_{20}$ (square), and $Tc_{40}$ and $Tc_{20}$ (circle).

[Figure 7] Figure 7 is a primary regression line of $Tc_5$ with respect to APTT by a percentage method.

[Figure 8] Figure 8 shows the slope (A), intercept (B), and correlation coefficient (C) of each of the primary regression lines of $Tc_5$ to $Tc_{95}$ with respect to APTT by a percentage method.

[Figure 9] Figure 9 shows errors in $Tc_5$ to $T_{C95}$ of 24 samples with respect to control (APTT by a percentage method). In the tables, the cells indicated by gray color indicate that the difference between the Tc and the control is within $\pm5\%$ (A) or $\pm3\%$ (B) of the control.

[Figure 10] Figure 10 shows A: a primary regression line of $Tc_{0.5}$ with respect to APTT by a percentage method; and B to D: the slope (B), intercept (C), and correlation coefficient (D) of primary regression lines of $Tc_{0.5}$ to $Tc_5$ with respect to APTT by a percentage method.

[Figure 11] Figure 11 shows errors in $Tc_{0.5}$ to $Tc_5$ of 24 samples with respect to control (APTT by a percentage method). In the table, the cells indicated by gray color indicate that the difference between the Tc and the control is within $\pm1\%$ of the control.

[Figure 12] Figure 12 shows examples of measured Te and Tc, and PT by a percentage method. A: PT minimum sample, and B: PT maximum sample. In the graphs, P(i) (the above) and V(i) (the below) are shown together with the point of time of Pmax (rhombus), PT (triangle) by a percentage method based on Pmax, $Te_{40}$ and $Te_{15}$ (square), and $Tc_{40}$ and $Tc_{15}$ (circle).

[Figure 13] Figure 13 is a primary regression line of $Tc_5$ with respect to PT by a percentage method.

[Figure 14] Figure 14 shows the slope (A), intercept (B), and correlation coefficient (C) of each of the primary regression lines of $Tc_5$ to $T_{C95}$ with respect to PT by a percentage method.

[Figure 15] Figure 15 shows errors in $Tc_5$ to $T_{C95}$ of 23 samples with respect to control (PT by a percentage method). In the tables, the cells indicated by gray color indicate that the difference between the Tc and the control is within $\pm5\%$ (A) or $\pm3\%$ (B) of the control.

[Figure 16] Figure 16 shows examples of measured Te and Tc, and coagulation time by a percentage method. A: [Fbg] minimum sample, and B: [Fbg] maximum sample. In the graphs, P(i) (the above) and V(i) (the below) are shown together with the calculation start point (rhombus) by an integrated value ratio, the coagulation time (triangle) by a percentage method based on the calculation start point, $Te_{75}$ and $Te_{30}$ (square), and $Tc_{75}$ and $Tc_{30}$ (circle).

[Figure 17] Figure 17 shows a primary regression line of [Fbg] arithmetic value of $Tc_{30}$ with respect to [Fbg] arithmetic value by a percentage method.

[Figure 18] Figure 18 shows the slope (A), intercept (B), and correlation coefficient (C) of each of the primary regression lines of [Fbg] arithmetic values based on $Tc_5$ to $Tc_{95}$ with respect to [Fbg] arithmetic values by a percentage method.

[Figure 19] Figure 19 shows errors in [Fbg] arithmetic values based on $Tc_5$ to $T_{C95}$ regarding Fbg concentration series sample data of 20 samples (10 samples $\times$ duplicate measurements) with respect to expected value. In the tables, the cells indicated by gray color indicate that the error of the arithmetic value from the expected value is within $\pm10\%$ (A) or within $\pm5\%$ (B).

[Figure 20] Figure 20 shows graphs of P(i) and V(i) (the left), P(i) and Z(i) (the middle), and enlarged Z(i) (the right) of samples A (the above) and B (the below).

[Figure 21] Figure 21 shows distributions of Vmax (A), Kmax (B), and Wmax (C) in samples used in Example 1 (APTT), Example 2 (PT), and Example 3 (Fbg). In the graphs, the dotted lines indicate fixed values of Vs(A), Ks(B), and Ws(C), and the solid lines indicate function Vs(A), function Ks(B), and function Ws(C).

[Figure 22] Figure 22 shows relationships between V(t) and Vs (the above), K(t) and Ks (the middle), and W(t) and Ws (the below) in the sample (A) with the smallest APTT and the sample (B) with the largest APTT in Example 1.

Detailed Description of the Invention

In a blood coagulation test, a prescribed reagent is added to a blood specimen, a subsequent blood coagulation reaction is measured, and blood coagulation time is measured from the coagulation reaction. In the present specification below, a blood specimen may be referred to as a sample. As the measurement of a blood coagulation reaction, a general method, for example, an optical method that measures the scattered light intensity, transmittance, absorbance, or the like, or a mechanical method that measures the viscosity of plasma is used.

The coagulation reaction curve of a normal sample depends on the measurement method, but basically shows a sigmoid shape. For example, a coagulation reaction curve based on the scattered light intensity of a normal sample is usually sharply increased by progression of coagulation at a point of time when a certain amount of time has passed since the addition of the reagent, and then reaches the plateau as the coagulation reaction approaches the end. In contrast, the coagulation reaction curves of abnormal samples with coagulation disorder show various shapes depending on the cause of the abnormality, such as delay of the curve rise time and a gradual increase. The variety in the coagulation reaction curve of an abnormal sample makes accurate measurement of coagulation time in an automated analyzer difficult.

In conventional general measurement of blood coagulation time, data at least until the end of coagulation reaction are acquired, and the coagulation time is calculated based on the acquired data. For example, in the case of calculating coagulation time based on the amount of scattered light, there are, for example, a procedure of judging a point of time at which the scattered light intensity is saturated as the end of coagulation reaction, and then determining a point of time at which the coagulation reaction curve reaches the maximum velocity or 1/N thereof during from the time of addition of the reagent until the time of the end of coagulation reaction as the coagulation time (a differentiation method), and a procedure of determining a point of time at which the scattered light intensity reaches 1/N of the amount of scattered light at the end of coagulation reaction as the coagulation time (a percentage method, see Patent Literature 1). However, false detection of the peak of a coagulation reaction velocity or the end of coagulation reaction is caused by abnormal shapes and noises of coagulation reaction curves of abnormal samples as described above, and, for example, the peak of a reaction velocity and the reaction end are detected at a too early point of time in some cases. Such false detection leads to calculation of inaccurate coagulation time.

Since an automated analyzer efficiently analyzes a large number of samples, it is desirable to promptly end the measurement after acquisition of necessary data of one sample and to start measurement of a next sample. However, such a procedure has a risk of inviting the end of measurement too early due to the above-described false detection of the end of coagulation reaction at a too early point of time and causing missing of necessary data. On the other hand, if the coagulation reaction measurement time for one sample is fixed as a sufficiently long time, it is possible to prevent data missing due to false detection of the end of coagulation reaction. However, such a procedure makes the measurement time of a large number of samples unnecessarily long and thereby reduces the total analysis efficiency.

The present invention can prevent false detection of the coagulation time resulting from the abnormal shape of a coagulation reaction curve as described above to allow accurate coagulation time measurement. In addition, according to the present invention, since minimum necessary coagulation reaction measurement time can be applied to individual coagulation time measurements of various blood specimens including a normal sample and an abnormal sample, the analysis time for one sample can be shortened.

[Method for measuring blood coagulation time]

1. Measurement of coagulation reaction

The present invention relates to a method for measuring the blood coagulation time of a blood specimen. The method for measuring blood coagulation time of the present invention (hereinafter, also referred to as the method of the present invention) measures the coagulation reaction of a subject blood specimen mixed with a reagent. The blood coagulation time is measured based on the data in the time series of the coagulation reaction obtained by this measurement. Examples of the blood coagulation time to be measured by the method of the present invention include prothrombin time (PT), activated partial thromboplastin time (APTT), and coagulation time in fibrinogen (Fbg) concentration measurement. In the present specification below, the method of the present invention will be mainly described using activated partial thromboplastin time (APTT) as an example of the coagulation time. The method of the present invention can be changed to another coagulation time (for example, prothrombin time (PT)) by those skilled in the art.

In the method of the present invention, as the subject blood specimen, plasma of a subject is preferably used. An anticoagulant that is usually used in a coagulation test may be added to the sample. For example, plasma is obtained by sampling blood with a blood collection tube containing sodium citrate and then performing centrifugation.

A coagulation time measurement reagent is added to the subject specimen to start blood coagulation reaction. The coagulation reaction of the mixed solution prepared by addition of the reagent can be measured. The coagulation time measurement reagent to be used can be arbitrarily selected depending on the measurement purpose. The reagents for measuring various types of coagulation time are commercially available (for example, APTT reagent Coagpia APTT-N, manufactured by Sekisui Medical Co., Ltd.). As the measurement of coagulation reaction, a general method, for example, an optical method that measures the amount of scattered light, transmittance, absorbance, or the like, or a mechanical method that measures the viscosity of plasma may be used. The point of time of starting coagulation reaction can be typically defined as the point of time at which the coagulation reaction is started by adding a reagent to a sample, but another timing may be defined as the point of time of starting the reaction. The time for continuing the measurement of coagulation reaction may be, for example, from several tens of seconds to about 7 minutes since the point of time of mixing a sample and a reagent. This measurement time may be an arbitrarily determined fixed value or may be until the point of time at which the end of coagulation reaction of each sample is detected. Furthermore, in the method of the present invention, measurement of a sample may be ended at a point of time at which the coagulation time of the sample is determined before the completion of coagulation reaction, and measurement of a next sample may be started. During the measurement time, measurement of the state of progress of the coagulation reaction (in the case of optical detection, photometry) is repeated at predetermined intervals. For example, measurement may be performed at 0.1 second intervals. The temperature of the mixed solution during the measurement is, for example, 30°C or more and 40°C or less under normal conditions, preferably 35°C or more and 39°C or less. Various conditions of measurement can be appropriately set depending on the subject specimen, reagent, measurement methods, and so on.

A series of procedure in the above-described measurement of coagulation reaction can be performed using an automated analyzer. An example of the automated analyzer is automated blood coagulation analyzer CP3000 (manufactured by Sekisui Medical Co., Ltd.). Alternatively, the procedure may be partially manually performed. For example, preparation of subject specimens is performed by a human, and the subsequent procedure can be performed with an automated analyzer.

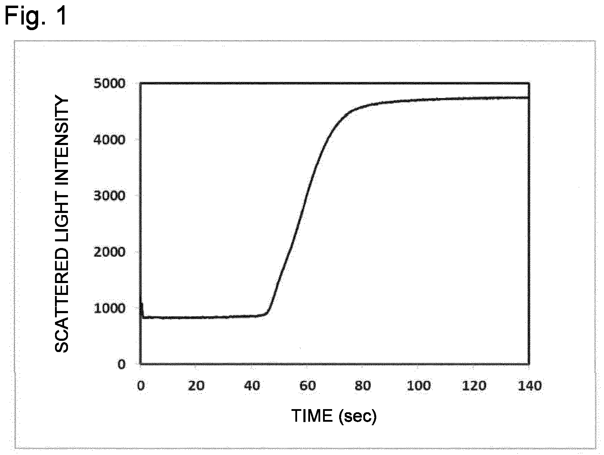

Figure 1 shows an example of measurement data. The horizontal axis of Figure 1 represents the time (coagulation reaction time) passed after addition of a calcium chloride solution (the point of time of start of reaction), and the vertical axis represents the amount of scattered light. Since the coagulation reaction of the mixed solution progresses, the scattered light intensity increases with time. In the present specification, such a curve showing the change in the amount of coagulation reaction with respect to the time of the coagulation reaction is referred to as a coagulation reaction curve.

A coagulation reaction curve based on the amount of scattered light as shown in Figure 1 is usually sigmoidlike. On the other hand, a coagulation reaction curve based on the amount of transmitted light is usually inverse sigmoidlike. In the present specification below, data analysis using a coagulation reaction curve based on the amount of scattered light will be described.

2. Data analysis

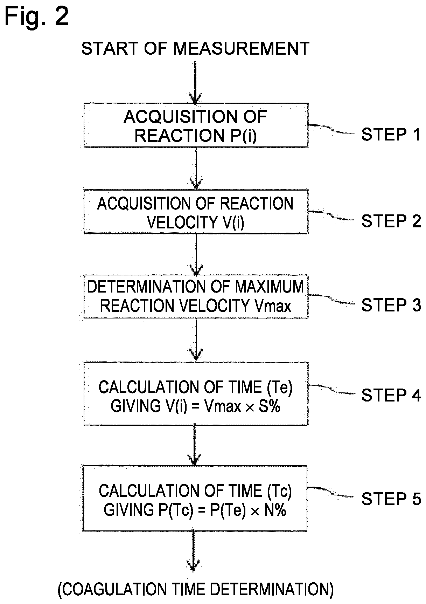

The present invention will now be described with reference to the basic flow of an embodiment of the method for measuring blood coagulation time according to the present invention shown in Figure 2.

In the method of the present invention, the measurement data R(i) (measured values of the amount of scattered light) about coagulation reaction measured from the point of time of starting reaction is successively acquired by an analyzer. Here, "i" represents the measuring point number. For example, when the measuring (photometric) interval is 0.1 seconds, the R(i) represents the measured value after $0.1 \times i$ seconds since the start of measurement.

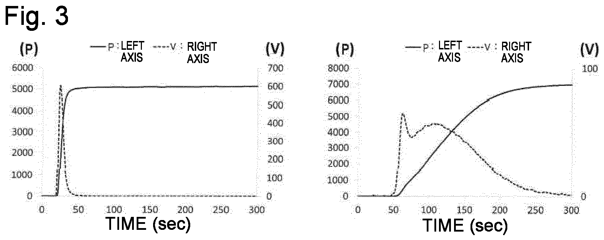

Since the measurement data R(i) includes noises at the time of measurement and a fluctuation unrelated to the reaction appearing immediately after the start of light measurement, the measured values are subjected to smoothing processing by a known method. When photometry of a coagulation reaction is performed using the amount of scattered light, zero point adjustment treatment, which is subtraction of the amount of scattered light derived from the reaction solution before the reaction, is performed. Accordingly, the acquired measurement data R(i) are successively subjected to smoothing processing and zero point adjustment to acquire reaction P(i) (step 1). The smoothing processing of measurement data can use any of various known methods relating to noise removal. Examples of the smoothing processing include filtering processing and processing by determining the difference value or the differential value by calculation of an average slope within the intervals described later and then integrating the values. In the zero point adjustment, for example, the smoothed measured value may be adjusted such that the value at the point of time of starting measurement is 0. Furthermore, the measurement data R(i) may be subjected to initial fluctuation removing treatment. The initial fluctuation removing treatment may be performed such that all values during from the time of starting photometry until previously determined time of removing initial fluctuation are 0. Basically, as shown in Figure 3, the reaction P(i) constitutes a coagulation reaction curve subjected to smoothing and zero point adjustment.

Reaction velocity V(i) is acquired from the determined reaction P(i) (step 2). V(i) is obtained by primary differentiation of P(i). The differentiation treatment can be performed by an arbitrary procedure, for example, by calculation of the average slope value within the intervals. In the calculation of the average slope value within the intervals, a certain number of measuring points before and after each measuring point i, for example, (2K + 1) measuring points from i - K to i + K can be used. For example, 5 measuring points: the points of i - 2, i - 1, i, i + 1, and i + 2, can be used. The average slope value means the slope value obtained by linear approximation of these plurality of measuring points. As the calculation method of the linear approximation, a usual method, such as a least-squares method, can be used. The average slope value of these measuring points can be recognized as the primary differential value at the measuring point i. In the present specification below, P(i) and V(i) may be simply abbreviated to P and V, respectively.

The relative differences between the shapes of reaction P and shapes of reaction velocity V of a normal sample having normal APTT and a factor VIII (FVIII) deficiency sample as an example having elongated APTT (hereinafter, elongation sample) will be described with reference to Figure 3. Figure 3 shows the normal sample in the left and the elongation sample in the right. In the horizontal axis, the time is converted into the time from the point of time of starting reaction. In the normal sample (left), the reaction P has an early rising point and a large rising slope, and the reaction velocity V has a high peak top position and an almost left-right symmetric shape. However, in the elongation sample (right), the reaction P has a late rising point and a small rising slope, and the reaction velocity V has a low peak top position (in this example, lower than 1/6 of the normal sample) and a left-right asymmetric shape, a bimodal shape, not having a single peak.

The method of the present invention calculates coagulation time Tc using the values of the above-described reaction P(i) and reaction velocity V(i). The method of the present invention can acquire P(i) and V(i) in parallel to the coagulation reaction measurement process.

First, the maximum reaction velocity Vmax is determined based on V(i) acquired so far (step 3). The Vmax most simply represents the maximum value of V(i) acquired so far. On the other hand, on the occasion of determining the Vmax, it is necessary to remove noises from the measuring instrument, which are observed in the coagulation reaction initial stage, and the influence by initial abnormal reaction (so-called early reaction). Accordingly, the Vmax is preferably the value of the peak top of the maximum peak after V(i) exceeded a prescribed threshold for removing noises.

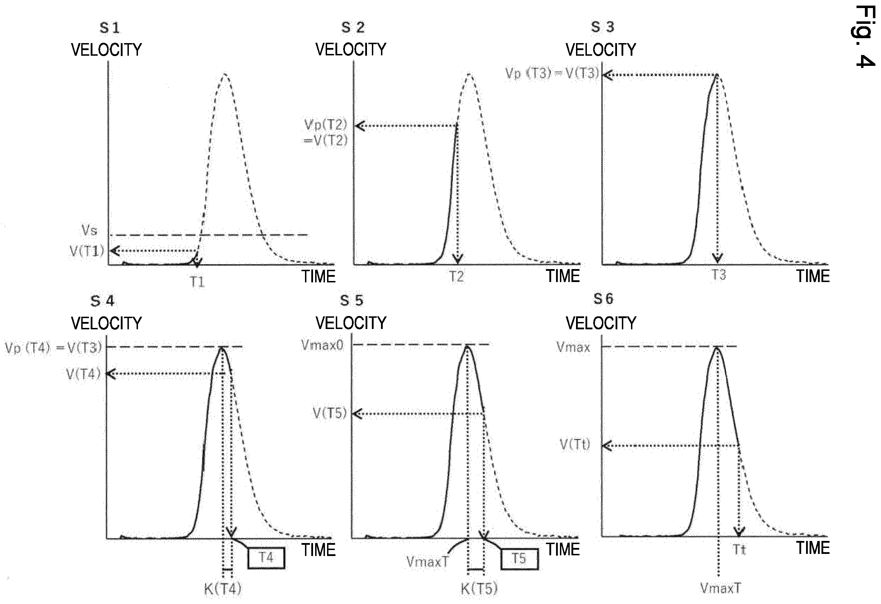

Preferably, in the method of the present invention, when a peak of successively obtained V(i) has a prescribed width, the value of the peak top of the peak is determined as Vmax. Figure 4 shows a conceptual scheme of an embodiment of the Vmax determination procedure. In Figure 4, S1 to S6 show, in the time series, V(i) acquired during from a point of time (T1) of the reaction initial stage to a point of time (Tt) of Vmax determination. In V(i) in each figure, the acquired data are represented by a solid line, and data that have not been obtained yet are represented by a short-dashed line, and eventually, V(i) forms a curve having a peak top (Vmax).

S1 shows V(i) until a point of time T1 immediately after the rising of coagulation reaction. The V(i) (= V(T1)) at T1 is not higher than the velocity threshold Vs. Vs is the velocity threshold for excluding initial noises. When V(i) exceeded Vs, research of Vmax is started.

S2 shows V(i) until a point of time T2 during increasing of the coagulation velocity. Vp is a tentative peak and represents the maximum value of V(i) acquired after V(i) exceeds the Vs until the latest measuring point. Vp is represented by the function Vp(t) at time t or the function Vp(i) of a measuring point.
The V(i) (= V(T2)) at T2 exceeds the Vs and is the maximum value of V(i) at the point of time T2 (that is, Vp(T2) = V(T2)), but does not arrive at the peak top yet.

S3 shows V(i) until the point of time T3 at which V(i) arrived at the peak top, where Vp(T3) = V(T3).

S4 shows V(i) until the point of time T4 after V(i) passes through the peak top and starts to decrease. The maximum value Vp(T4) of V(i) at T4 remains in the peak top (= V(T3)). K is the maximum value continuation width and represents the width (or number of measuring points) of the time during which Vp continues to be a constant value, i.e., to be the peak top (= V(T3)). K is represented by the function K(t) at time t or the function K(i) of a measuring point. For example, K(T4) in the figure represents K at the point of time T4 and corresponds to the time width from the point of time T3 of the peak top until time T4. K(i) at the point of time T4 is the value obtained by multiplying the K(T4) by the measurement frequency (number of measuring points/sec) of coagulation reaction measurement.

S5 shows V(i) further decreased until the point of time T5. Vp continues to be the peak top V(T3) until the point of time T5, and K(T5) exceeds the maximum value continuation width threshold Ks (that is, K(T5) > Ks).
On this occasion, Vp(T5) (= V(T3)) is determined as tentative maximum value VmaxO.

S6 shows V(i) at the detection end point Tt of Vmax. Tt is the point (the point of time or measuring point) at which V(i) decreased until a prescribed value, after the VmaxO detection, while K(i) > Ks is maintained. Tt in S6 is defined as a point of time at which V(i) is decreased to 50% of VmaxO, but can be arbitrarily set. Preferably, Tt is a point at which V(i) is 40% to 95% of VmaxO. VmaxO is determined as true maximum value Vmax at the point of time Tt, and the time satisfying V(i) = Vmax is determined as maximum value time VmaxT. On the other hand, after the VmaxO detection, if V(i) increases again without decreasing to the prescribed value and exceeds the VmaxO, K(i) is reset to 0, and S2 to S5 are carried out again to detect new VmaxO.

Table 1 shows the states of V(i) in S1 to S5.

[Table 1]

| Reaction state | S1 | S2 | S3 | S4 | S5 | S6 |
|---|---|---|---|---|---|---|
| Time: t (sec) | T1 | T2 | T3 | T4 | T5 | Tt |
| V(i) exceeded Vs or not? | no | yes | yes | yes | yes | yes |
| Tentative peak Vp(i) | - | V(T2) | V(T3) | V(T3) | V(T3) | V(T3) |

(continued)

| | | | | | | |
|---|---|---|---|---|---|---|
| Maximum value continuation width K(i) | - | 1 | 1 | (T4-T3)xf+1 | (T5-T3)xf+1 | (Tt-T3)xf+1 |
| K(i) exceeded Ks or not? | - | no | no | no | yes | yes |
| VmaxO | - | - | - | - | V(T3) | V(T3) |
| Vmax | - | - | - | - | - | V(T3) |
| f: measurement frequency (number of measuring points/sec) of coagulation reaction measurement | | | | | | |

When V(i) does not exceed the velocity threshold Vs within the measurement time, it is judged that there is no coagulation reaction. When V(i) exceeds the Vs within the measurement time but the maximum value continuation width K does not exceed the threshold Ks, it is judged that the coagulation reaction is not ended yet. Even if the detection end point Tt cannot be detected after detection of VmaxO and Vmax is not determined, it is judged that the coagulation reaction is not ended yet.

In another embodiment, Vmax can be determined based on the peak width W of V(i). The peak width W is the width (time or number of measuring points) between the latest point and the point at which the height of V(i) is X% of a tentative peak Vp before the latest point and before Vp. X is 1 or more and less than 100 and is preferably 40 to 95. X is preferably larger than parameter S that is used for calculating a calculation start point Te described later, but may be equal to or smaller than the S. In more detail, when the coagulation time to be measured is APTT, X is preferably 41 to 80, and when the coagulation time is PT, X is preferably 40 to 95. In the present specification, the peak width W may be called as "X% peak width" according to X as the reference. In this procedure, as in the above-described process, after V(i) exceeded Vs, Vp is successively detected, and the peak width W(t) at time t or the peak width W(i) at a measuring point i are determined. When the peak width W exceeds a peak width threshold Ws, Vp is determined as tentative maximum value VmaxO, and then at the point of time Tt (the definition is the same as above), the VmaxO is determined as true maximum value Vmax, and the time satisfying V(i) = Vp is determined as maximum value time VmaxT. Alternatively, more simply, Vp at Tt may be defined as true maximum value Vmax. However, when the peak width W does not exceed a threshold Ws within the measurement time, it is judged that the coagulation reaction is not ended yet.

As the velocity threshold Vs, the maximum value continuation width threshold Ks, and the peak width threshold Ws, fixed values previously determined can be respectively used. For example, appropriate Vs, Ks, and Ws can be set by measuring coagulation time for a reference sample pack including a normal sample and various types of abnormal samples and using the resulting data of V(i) for the pack. For example, Vs is set to a value smaller than the smallest Vmax in those of each sample determined from the measurement data of the reference sample pack. Regarding Ks and Ws, the maximum value continuation width K and peak width W of each sample of the reference sample pack were determined by the above process, and appropriate values may be set as Ks and Ws based on the determined K and W, respectively. Preferably, Ks is set to a value smaller than the smallest value of the maximum values of K(i) (hereinafter, referred to as Kmax) determined for each sample, and Ws is set to a value smaller than the smallest value of the maximum values of W(i) (hereinafter, referred to as Wmax) determined for each sample.

Alternatively, the velocity threshold Vs, the maximum value continuation width threshold Ks, and the peak width threshold Ws may be functions of time t or measuring point i. Even when Vs, Ks, and Ws are functions of t or i, the functions may be set based on the values of Vmax, Ks, and Ws determined from each sample of the reference sample pack, as in the above.

For example, Vs can be set as a function represented by a curve showing a value always smaller than Vmax on a graph plotting the Vmax determined from each sample against VmaxT. Similarly, Ks and Ws can be set as functions represented as curves showing values always smaller than Kmax and Wmax, respectively, on a graph plotting the Kmax and Wmax determined from each sample against VmaxT. In a preferable embodiment, functions Vs, Ks, and Ws are, for example, calculated by the process described in Reference Example 1 described later and can be determined by the following equations:

$$Vs = b + (a/t^2)$$

$$Ks = b' + (a' \times t^2)$$

$$Ws = b" + (a" \times t^2)$$

[a, a', a", b, b', and b" are coefficients, and t is time (sec)]

Alternatively, Vs, Ks, and Ws can be determined as functions of measuring point i by converting t in the above equations into i. Even when the reaction velocity V(i) is small or bimodal as often observed in abnormal samples, Vmax can be appropriately detected by using variable values as Vs, Ks, and Ws.

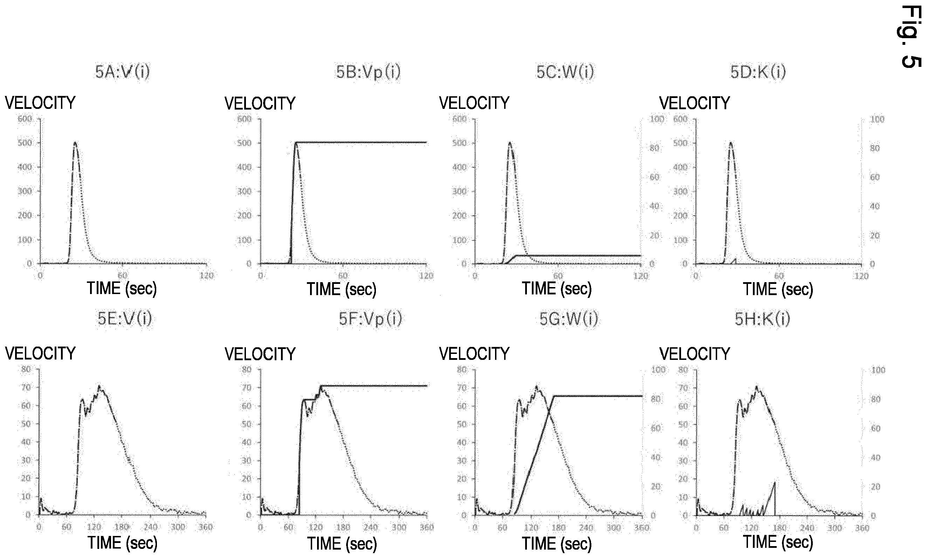

Figure 5 shows relationships between V(i) and Vp(i), W(i), or K (i) . A to D of Figure 5 show V(i) and relationships between V(i) and Vp(i), W(i), or K(i) in a normal sample with normal APTT. E to H of Figure 5 show V(i) and relationships between V(i) and Vp(i), W(i), or K(i) in an abnormal (FVIII deficiency) sample. In each graph, V(i) is indicated by an alternate long and short dash line until a point of time at which V(i), after Vmax, arrives at 70% of Vmax (detection end point Tt) and thereafter by a dotted line. Vp(i), W(i), and K(i) are indicated by a solid line. W(i) uses a 70% peak width. As shown in A to D of Figure 5, V(i) of the normal sample shows a unimodal peak, and Vp(i) increases until V(i) reaches the peak (Vmax) and is then constant. W(i) increases until the detection end point Tt and is then constant. K(i) increases until the detection end point Tt. On the other hand, V(i) of the abnormal sample shown in E to H of Figure 5 shows an approximately bimodal peak including a large number of small peaks, and the second peak is the maximum value Vmax. Vp(i) stepwise increases as V(i) reaches a higher peak and is constant after V(i) reached Vmax. W(i) increases until the detection end point Tt and is then constant. K(i) is reset to 0 at every time at which a higher V(i) peak appears, and thereby a peak of K(i) corresponding to a peak of V(i) appears.

After the determination of Vmax, a point Te at which V(i) is S% of Vmax (100%) after the time of VmaxT is determined (step 4). Te is a point of time (or measuring point) that is recognized such that the coagulation reaction proceeded to a considerable extent and is calculated based on a ratio of V(i) to maximum reaction velocity Vmax (hereinafter also referred to as "maximum velocity ratio"). Here, S% corresponds to a maximum velocity ratio, and VmaxT < Te. S can be determined within a range of larger than 0 and smaller than 100 and is preferably 1 to 95 and more preferably 5 to 95. The smaller S is, the better the measurement precision of the coagulation time tends to be, but the longer the required measurement time is. When the coagulation time to be measured is APTT, S is further preferably 5 to 40 and further preferably 5 to 20. When the coagulation time is PT, S is further preferably 5 to 40 and further preferably 5 to 15. In the method of the present invention, Te is used as the calculation start point of coagulation time.

Subsequently, a point of time Tc at which P(i) is N% of P(Te) (P(Tc) = P(Te) × N%) is determined using P(i) acquired until Te (step 5). The resulting Tc is determined as the coagulation time. N may be 0 < N < 100 and is preferably 10 to 70 and more preferably 20 to 60.

As shown in Examples below, the coagulation time Tc based on the maximum velocity ratio calculated by the method of the present invention has a high correlation with the coagulation time by a percentage method, which is a conventional common method (for example, a method for calculating the time for P(i) to reach a certain percentage of its maximum value Pmax as the coagulation time). Accordingly, the coagulation time of a subject specimen can be precisely determined by the method of the present invention. Furthermore, in the method of the present invention, coagulation time measurement that is unlikely to receive influences, such as measurement noise and early reaction, and has higher reliability is possible by the step of detecting a true peak of reaction velocity V(i) (step 3 described above).

Furthermore, in the method of the present invention, it becomes possible to determine the coagulation time when reaction velocity V(i) arrived at a prescribed threshold (Vmax × S%) after reaching maximum reaction velocity Vmax. Accordingly, in the method of the present invention, it is not necessary to continue the measurement until the coagulation reaction reaches the plateau, unlike the conventional percentage method. Furthermore, also when a large number of samples are analyzed by an automated analyzer, according to the method of the present invention, it is not necessary to set a long measurement time for an abnormal sample with low coagulability, unlike the conventional percentage method. Accordingly, according to the present invention, it is possible to reduce or optimize the analysis time of samples and thereby improve the analysis efficiency.

3. Measurement of coagulation factor concentration

A normal blood includes coagulation factors, such as coagulation factors I to XIII, and abnormality and deficiency of these coagulation factors lead to abnormality of coagulability. Generally, the coagulation factor concentration of a subject specimen can be measured using a dedicated reagent for each coagulation factor based on the coagulation time of a measurement sample prepared from the subject specimen. Accordingly, the coagulation factor concentration of the subject specimen can be measured using the coagulation time of the measurement sample measured by the method of the present invention. Generally, the coagulation factor concentration is measured based on a standard curve showing a relationship between coagulation time and coagulation factor concentration. Accordingly, the coagulation factor concentration of the subject specimen can be calculated by applying the coagulation time of the measurement sample measured by the method of the present invention to a standard curve made in advance. Preferable examples of the coagulation factor to be measured by the method of the present invention include factor I (fibrinogen), factor VIII, and factor IX.

In calculation of the coagulation time to be used in measurement of the coagulation factor concentration by the method of the present invention, the threshold ("Vmax × S%") of V(i) for determining the calculation start point Te based on the maximum velocity ratio is preferably a prescribed value set within a range of 1% to 95% (i.e., S = 1 to 95) of Vmax, more preferably 5% to 95% (i.e., S = 5 to 95) of Vmax, further preferably 20% to 95% (i.e., S = 20 to 95) of Vmax, and further preferably 30% to 75% (i.e., S = 30 to 75) of Vmax. In addition, as the X% peak width W for Vmax determination, it is preferable to use a peak width of from 75% to 95%. Here, X is preferably larger than S, but may be equal to or smaller than S.

4. Application to another coagulation reaction-measuring method

The method for measuring blood coagulation time of the present invention has been described above by the case of coagulation reaction measurement based on scattered light intensity as an example. However, the method of the present invention can be applied to a method for measuring blood coagulation time using another method for measuring coagulation reaction (for example, a method for measuring blood coagulation reaction based on transmittance, absorbance, viscosity, or the like) by those skilled in the art. For example, the positive and negative of reaction P(i) obtained from an inverse sigmoid-like coagulation reaction curve based on the amount of transmitted light or the like becomes reversed with respect to those based on the amount of scattered light described above. It is obvious to those skilled in the art that in such a case, the signs of P(i) and V(i) in the above-described steps 1 to 4 are reversed, for example, the minimum reaction velocity Vmin is determined instead of the maximum reaction velocity Vmax.

Examples

The present invention will now be described in further detail with examples, but is not limited to these examples.

Example 1: Measurement of coagulation time (APTT)

1. Method

1.1) Sample

As subject specimens, 9 normal plasma samples and 15 abnormal plasma samples with elongated APTT, 24 samples in total, were used. As the normal plasma, Normal Donor Plasma manufactured by CliniSys Associates, Ltd. was used. In the abnormal plasma, as coagulation factor deficiency plasma (factor deficient plasma), 2 samples each of factor FVIII deficiency plasma, factor FIX deficiency plasma, factor FXI deficiency plasma, and factor FXII deficiency plasma, 2 samples of lupus anticoagulant positive plasma (Lupus Anticoagulant Plasma), and 5 samples of unfractionated heparin-containing plasma (Anticoagulant Plasma) (all of them are manufactured by CliniSys Associates, Ltd.) were used.

1.2) Reagent

As the APTT reagent, Coagpia APTT-N (manufactured by Sekisui Medical Co., Ltd.) was used.

1.3) Coagulation reaction measurement

Coagulation reaction measurement was performed using an automated blood coagulation analyzer CP3000 (manufactured by Sekisui Medical Co., Ltd.). Each (50 μL) of the samples was dispensed in a cuvette (reaction container) and was then warmed at 37°C for 45 seconds, the APTT reagent (50 μL) warmed to about 37°C was then added to the cuvette, and, after 171 seconds, a calcium chloride solution (50 μL) was further added thereto to start coagulation reaction. The reaction was performed while keeping the temperature about 37°C. The measurement (photometry) of coagulation reaction was performed by irradiating the cuvette with light having a wavelength of 660 nm from an LED light as a light source and measuring the amount of 90-degree side scattered light at 0.1 seconds intervals. The maximum measurement time was 360 seconds (number of data: 3600, 0.1 seconds intervals).

1.4) Acquisition of reaction P(i) and reaction velocity V(i)

Reaction P(i) was produced by subjecting the photometric data from each sample to smoothing processing including noise removal and then performing zero point adjustment treatment such that the scattered light intensity at the point of time of starting photometry is 0. The primary differential value V(i) was calculated from the P(i).

1.5) APTT measurement (percentage method)

The APTT of each sample was measured by a percentage method. That is, the point of time at which P(i) arrived at the maximum value Pmax within the measurement time was determined as the coagulation reaction end point, and the point of time at which P(i) reached 50% of the amount of scattered light at the coagulation reaction end point was determined as APTT. Table 2 shows the category and number of subject specimens and the minimum and maximum values of APTT in the samples of each category.

[Table 2]

| Sample category | Number of samples | APTT (sec) Minimum value | APTT (sec) Maximum value |
|---|---|---|---|
| Normal plasma | 9 | 27.2 | 35.4 |
| FVIII deficient plasma | 2 | 38.3 | 141.2 |
| FIX deficient plasma | 2 | 34.2 | 75.1 |
| FXI deficient plasma | 2 | 42.3 | 87.6 |
| FXII deficient plasma | 2 | 35.1 | 81.0 |
| LA positive plasma | 2 | 55.0 | 78.4 |
| Heparin-containing plasma | 5 | 38.2 | 126.2 |

1.6) Calculation of coagulation time Tc based on maximum velocity ratio

Maximum reaction velocity Vmax was detected from V(i) obtained for each sample. The tentative maximum value VmaxO when the maximum value continuation width K(i) exceeded the maximum value continuation width threshold Ks and then reached the detection end point Tt (the point of time at which V(i) became 70% of Vmax) was determined as Vmax. The velocity threshold Vs was determined as a function represented by the following equation according to the process of Reference Example 1 described later. Ks was set to 2.2.

$$Vs(i) = -83 + (158214/i^2) \text{ [i represents a measuring point].}$$

Time Te giving V(i) = Vmax × S% was determined (S = 0.5, 1, 1.5, 2, 2.5, 3, 3.5, 4, 4.5, 5, 10, 15, 20, 25, 30, 35, 40, 45, 50, 55, 60, 65, 70, 75, 80, 85, 90, and 95). Time Tc giving P(Tc) = P(Te) × 50% was determined as coagulation time. The Te and Tc determined for each S are referred to as Tes and Tcs, respectively, according to the corresponding S. For example, Te and Tc when S is 5 are referred to as $Te_5$ and $Tc_5$, respectively.

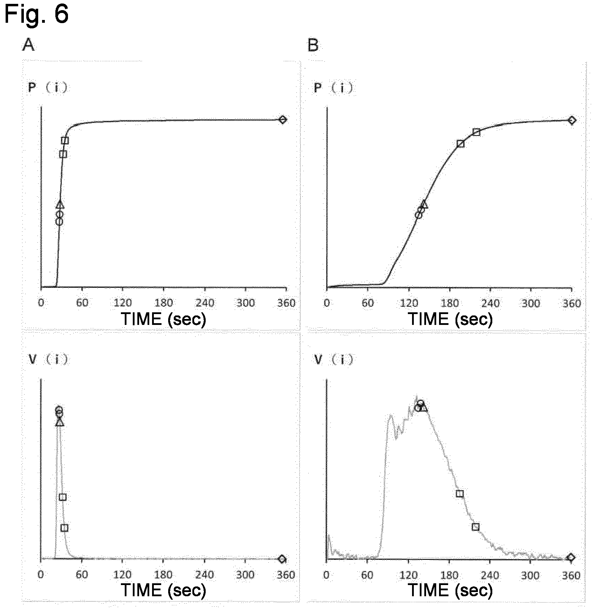

Figure 6 shows examples of measured Te and Tc, and APTT by a percentage method. A and B of Figure 6 show the P(i) (the above) and V(i) (the below) of samples of which the APTT by a percentage method were minimum (27.2 sec) and maximum (141.2 sec), respectively, together with the point of time of Pmax (rhombus), APTT (triangle) by a percentage method based on Pmax, $Te_{40}$ and $Te_{20}$ (square), and $Tc_{40}$ and $Tc_{20}$ (circle). In both A and B of Figure 6, $Tc_{40}$ and $Tc_{20}$ marked with circles were close to APTT, marked with a triangle, by a percentage method (conventional method) based on Pmax, and it was suggested that they reflect APTT. In addition, in both A and B of Figure 6, the time taken for P(i) to become the maximum value Pmax was about 360 seconds, and the differences between the time of Pmax and the time of $Te_{40}$ or $Te_{20}$ were about 320 seconds in A of Figure 6 and were about 140 to 160 seconds in B of Figure 6. It was shown that APTT can be calculated 140 seconds or more faster compared to the known percentage method based on Pmax by determining Te and Tc based on the maximum velocity ratio.

2. Evaluation of precision of Tc

2.1) Correlation analysis

The correlation between APTT by a percentage method and APTT(Tc) based on the maximum velocity ratio was evaluated. Each Tc ($Tc_5$ to $Tc_{95}$) when S was 5 to 95 was subjected to primary regression analysis with APTT by a percentage method, and the slope, intercept, and correlation coefficient of the regression line were determined.

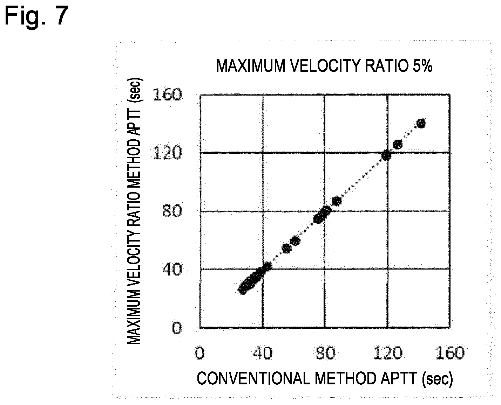

Figure 7 shows a primary regression line of $Tc_5$ with respect to APTT by a percentage method for 24 samples.

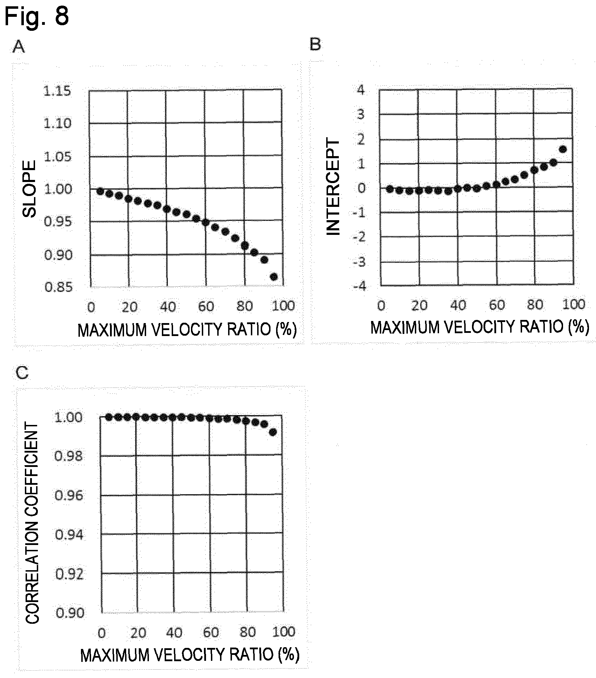

$Tc_5$ had a high correlation with APTT by a percentage method. The correlation between APTT by a percentage method and Tc was evaluated. Each Tc ($Tc_5$ to $Tc_{95}$) when S was 5 to 95 was subjected to primary regression analysis with APTT by a percentage method, and the slope, intercept, and correlation coefficient of the regression line were determined. Figure 8 shows the slopes, intercepts, and correlation coefficients of the primary regression lines of $Tc_5$ to $Tc_{95}$ with respect to APTT by a percentage method. In the range of $Tc_5$ to $Tc_{95}$, the slopes of the regression lines were 0.86 to 1.00, the intercepts were -0.1 to 1.6, and the correlation coefficients were 0.992 to 1.000 (A to C of Figure 8).

It was confirmed that the APTT measuring method based on maximum velocity ratio has performance satisfying In-vitro diagnostic drug approval criteria, "comparing to control measurement method, the correlation coefficient is 0.9 or more, and the slope of regression line equation is 0.9 to 1.1", by the Ministry of Health, Labour and Welfare.

2.2) Accuracy of measurement

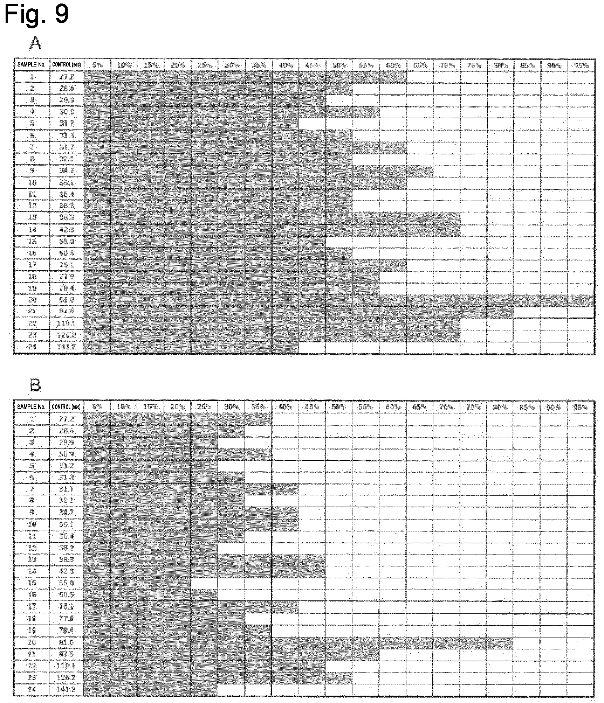

The $Tc_5$ to $T_{C95}$ of 24 samples were compared to a control (APTT by a percentage method). Each row of the tables of A and B of Figure 9 represents Tc (from the left, $Tc_5$ to $Tc_{95}$) of each sample. When the difference between Tc and the control is within ±5% (A of Figure 9) and ±3% (B of Figure 9) of the control, it is represented by gray color. $Tc_5$ to $Tc_{40}$ of all samples agreed with the control within an error of ±5%, and $Tc_5$ to $Tc_{20}$ of all samples agreed with the control within an error of ±3%.

3. Evaluation-2 of precision of Tc

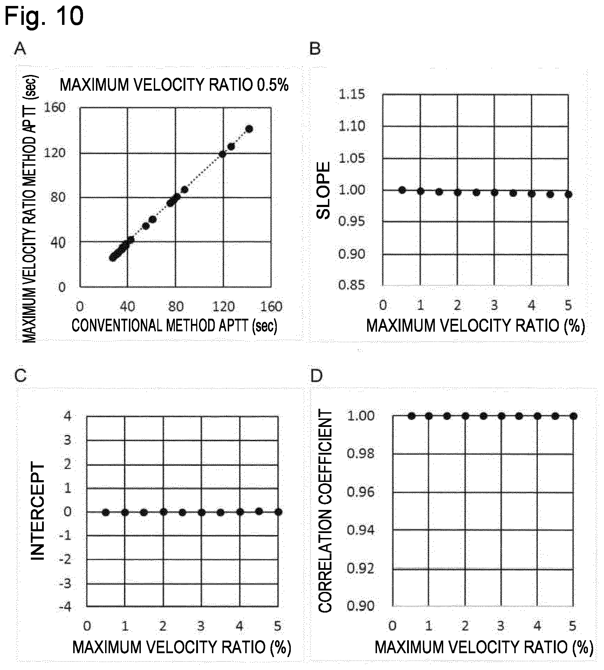

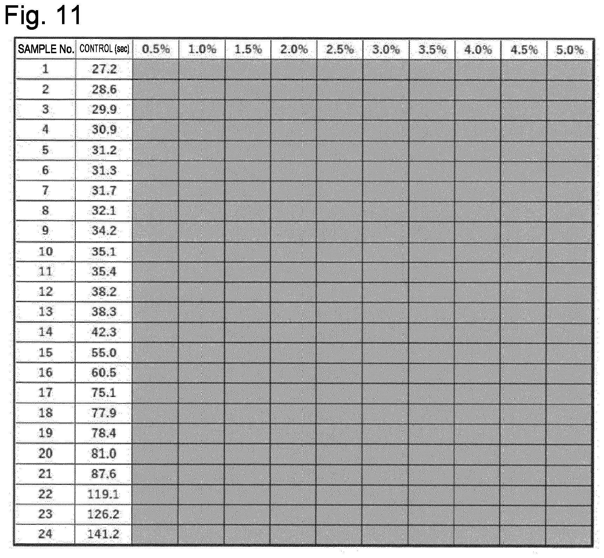

Each of $Tc_{0.5}$ to $Tc_5$ was subjected to primary regression analysis with APTT by a percentage method by the same process as in 2.1) above, and the slope, intercept, and correlation coefficient of the regression line were determined. The primary regression line of $Tc_{0.5}$ with respect to APTT by a percentage method for 24 samples is shown in A of Figure 10, and the slopes, intercepts, and correlation coefficients of the primary regression lines of $Tc_{0.5}$ to $Tc_5$ with respect to APTT by a percentage method are shown in B to D, respectively, of Figure 10. All of $Tc_{0.5}$ to $Tc_5$ had very high correlations with APTT by a percentage method. Figure 11 is a table comparing $Tc_{0.5}$ to $Tc_5$ with a control (APTT by a percentage method) for 24 samples by the same process as in 2.2). When the difference between Tc and the control is within ±1% of the control, it is represented by gray color. $Tc_{0.5}$ to $Tc_5$ of all samples agreed with the control within an error of ±1%.

Example 2: Coagulation time (PT) measurement

1. Method

1.1) Sample

As subject specimens, nine normal plasma samples and 14 abnormal plasma samples with elongated PT, 23 samples in total, were used. As the normal plasma, plasma of normal subjects was used. As the abnormal plasma, plasma of patients administered with warfarin as an anticoagulant and having a PT-INR value, which indicates blood warfarin concentration, of 1 to 2 (five samples), of 2 to 3 (five samples), and of 3 to 4 (four samples) was used.

1.2) Coagulation reaction measurement

The coagulation reaction measurement was performed using an automated blood coagulation analyzer CP3000 (manufactured by Sekisui Medical Co., Ltd.). Each (50 μL) of the samples was dispensed in a cuvette (reaction container) and was then warmed at 37°C for 45 seconds, and a thromboplastin solution (100 μL) warmed to about 37°C was then added to the cuvette to start coagulation reaction. The reaction was performed while keeping the temperature about 37°C. The measurement (photometry) of coagulation reaction was performed by irradiating the cuvette with light having a wavelength of 660 nm from an LED light as a light source and measuring the amount of 90-degree side scattered light at 0.1 seconds intervals. The maximum measurement time was 300 seconds (number of data: 3000, 0.1 seconds intervals).

1.3) Reaction P(i) and acquisition of reaction velocity V(i)

Reaction P(i) was produced by subjecting the photometric data from each sample to smoothing processing including noise removal and then performing zero point adjustment treatment such that the scattered light intensity at the point of time of starting photometry is 0. The primary differential value V(i) was calculated from the P (i) .

1.4) PT measurement (percentage method)

The PT of each sample was measured by a percentage method. The point of time at which P(i) arrived at the maximum value Pmax within the measurement time was determined as the coagulation reaction end point, and the point of time at which P(i) arrived at 45% of the amount of scattered light at the coagulation reaction end point was determined as PT. Table 3 shows the category and number of subject specimens and the minimum and maximum values of PT in the samples of each category.

[Table 3]

| Sample category | Number of samples | PT (sec) Minimum value | PT (sec) Maximum value |
|---|---|---|---|
| Normal plasma | 9 | 11.4 | 12.1 |
| PT-INR 1-2 | 5 | 9.5 | 23.4 |
| PT-INR 2-3 | 5 | 20.6 | 28.6 |
| PT-INR 3-4 | 4 | 31.3 | 51.6 |

1.5) Calculation of coagulation time Tc based on maximum velocity ratio

Maximum reaction velocity Vmax was detected from the V(i) obtained for each sample by the same process as in Example 1. The velocity threshold Vs was determined as a function represented by the following equation according to the process of Reference Example 1 described later. Ks was set to 0.8.

$$\mathtt{Vs(i) = -148 + (51935/i^2) \ [i \ represents \ a \ measuring}$$

$$\mathtt{point]}.$$

Time Te giving V(i) = Vmax × S% and time Tc giving P(Tc) = P(Te) × 45% were determined (S = 5, 10, 15, 20, 25, 30, 35, 40, 45, 50, 55, 60, 65, 70, 75, 80, 85, 90, and 95), and $Tc_5$ to $Tc_{95}$ were obtained.

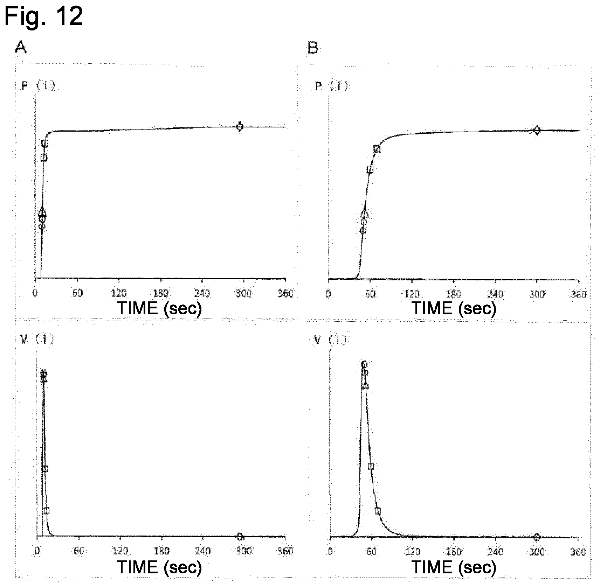

Figure 12 shows examples of measured Te and Tc and PT by a percentage method. A and B of Figure 12 show the P(i) (the above) and V(i) (the below) of samples of which the PT by a percentage method were minimum (9.5 sec) and maximum (51.6 sec), respectively, together with the point of time of Pmax (rhombus), PT (triangle) by a percentage method based on Pmax, $Te_{40}$ and $Te_{15}$ (square), and $Tc_{40}$ and $Tc_{15}$ (circle) . In both A and B of Figure 12, $Tc_{40}$ and $Tc_{15}$ marked with circles were close to PT, marked with a triangle, by a percentage method (conventional method) based on Pmax, and it was suggested that they reflect PT. In addition, in both A and B of Figure 12, the time taken for P(i) to become the maximum value Pmax was about 300 seconds, and the differences between the time of Pmax and the time of $Te_{40}$ or $Te_{15}$ were about 285 seconds in A of Figure 12 and were about 240 to 250 seconds in B of Figure 12. It was shown that PT can be calculated 240 seconds or more faster compared to the conventional percentage method based on Pmax by determining Te and Tc based on the maximum velocity ratio.

2. Evaluation of precision of Tc

2.1) Correlation analysis

Each of $Tc_5$ to $T_{C95}$ was subjected to primary regression analysis with PT measured by a percentage method by the same process as in 2.1) of Example 1, and the slope, intercept, and correlation coefficient of each regression line were determined.

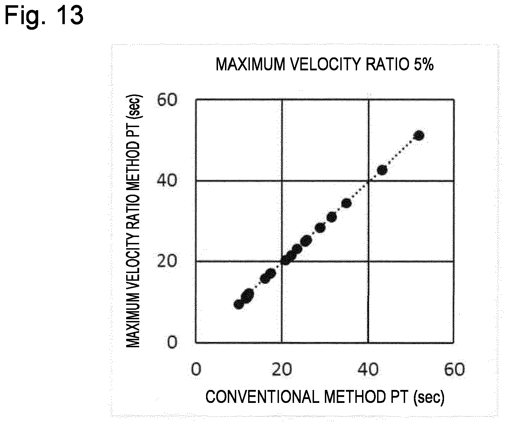

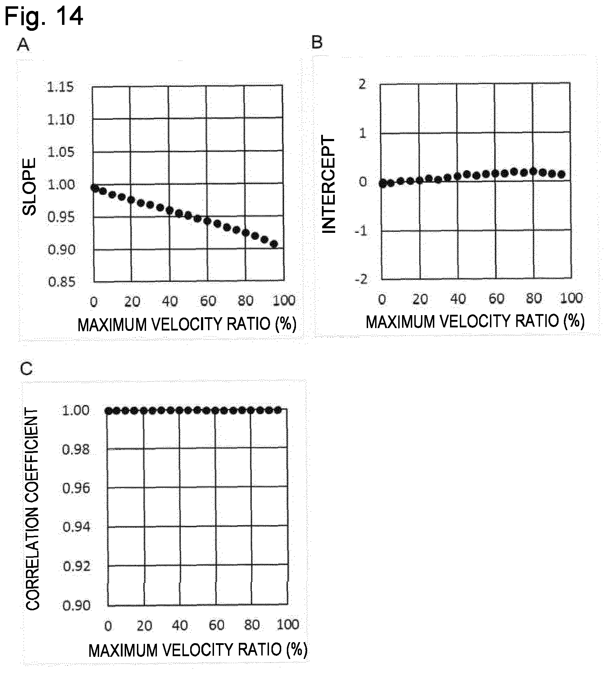

Figure 13 shows a primary regression line of $Tc_5$ with respect to PT by a percentage method for 23 samples. $Tc_5$ had a high correlation with PT by a percentage method. The correlation between PT by a percentage method and Tc was evaluated. Each Tc ($Tc_5$ to $Tc_{95}$) when S was 5 to 95 was subjected to primary regression analysis with PT by a percentage method, and the slope, intercept, and correlation coefficient of each regression line were determined. Figure 14 shows the slopes, intercepts, and correlation coefficients of the primary regression lines of $Tc_5$ to $T_{C95}$ with respect to PT by a percentage method. In the range of $Tc_5$ to $Tc_{95}$, the slopes of the regression lines were 0.91 to 1.00, the intercepts were 0.0 to 0.2, and the correlation coefficients were all 1.000 (A to C of Figure 14). It was confirmed that the PT measuring method based on maximum velocity ratio has performance satisfying In-vitro diagnostic drug approval criteria, "comparing to control measurement method, the correlation coefficient is 0.9 or more, and the slope of regression line equation is 0.9 to 1.1", by the Ministry of Health, Labour and Welfare.

2.2) Accuracy of measurement

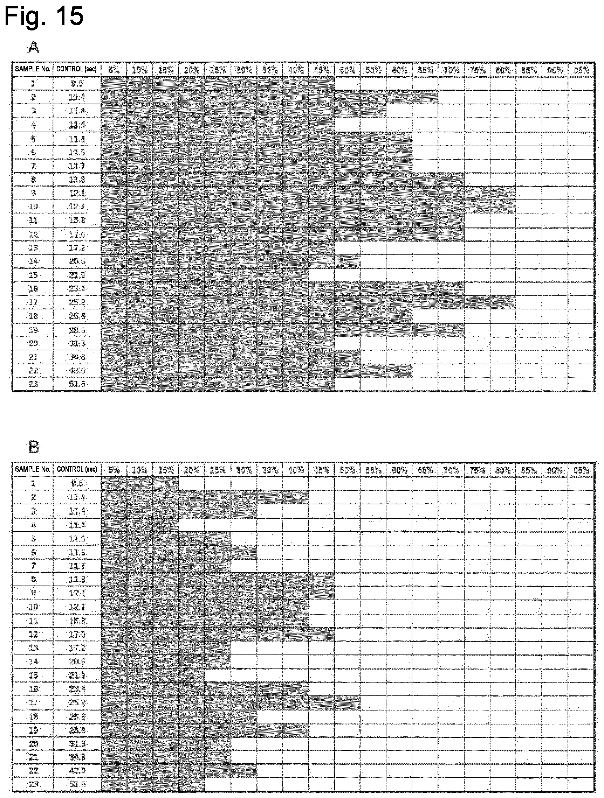

The $Tc_5$ to $Tc_{95}$ of 23 samples were compared to a control (PT by a percentage method). Each row of the tables of A and B of Figure 15 represents Tc (from the left, $Tc_5$ to $Tc_{95}$) of each sample. When the difference between Tc and the control is within ±5% (A of Figure 15) or ±3% (B of Figure 15) of the control, it is represented by gray color. $Tc_5$ to $Tc_{40}$ of all samples agreed with the control within an error of ±5%, and $Tc_5$ to $Tc_{15}$ of all samples agreed with the control within an error of ±3%.

Example 3: Fibrinogen concentration measurement

1. Method

1.1) Sample

Human fibrinogen (Human Fibrinogen manufactured by Enzyme Research Laboratories, Product name: FIB 2) was added to human fibrinogen-removed plasma (Fibrinogen Deficient Human Plasma manufactured by Affinity Biologicals Inc., Product name: Fg Deficient Plasma) to produce a sample (sample 10) with a fibrinogen concentration ([Fbg]) of 980 mg/dL. As standard samples for producing a standard curve, the sample 10 and a physiological saline solution were mixed at volume ratios of 1 : 9, 7 : 3, and 10 : 0 to prepare three samples with fibrinogen concentration ([Fbg]) of 98 mg/dL, 686 mg/dL, and 980 mg/dL, respectively. Separately, the sample 10 and the human fibrinogen-removed plasma were mixed at volume ratios of 1 : 9 to 10 : 0 to prepare ten concentration series samples with stepwise different fibrinogen concentrations ([Fbg]) (Table 4).

[Table 4]

| Sample | Dilution ratio | [Fbg] (mg/dL) |
|---|---|---|
| 1 | 1:9 | 98 |
| 2 | 2:8 | 196 |
| 3 | 3:7 | 294 |
| 4 | 4:6 | 392 |
| 5 | 5:5 | 490 |
| 6 | 6:4 | 588 |
| 7 | 7:3 | 686 |
| 8 | 8:2 | 784 |
| 9 | 9:1 | 882 |
| 10 | 10:0 | 980 |

1.2) Coagulation reaction measurement

As the fibrinogen measurement reagents, the thrombin reagent and sample diluent included in Coagpia Fbg (manufactured by Sekisui Medical Co., Ltd.) were used. The coagulation reaction measurement was performed using an automated blood coagulation analyzer CP3000 (manufactured by Sekisui Medical Co., Ltd.). A sample (10 μL) and the sample diluent (90 μL) were dispensed in a cuvette and were warmed at 37°C for 45 seconds, and a thrombin reagent (50 μL) warmed to about 37°C was then added to the cuvette to start coagulation reaction. The reaction was performed while keeping the temperature about 37°C. The coagulation reaction measurement was performed by irradiating the cuvette with light having a wavelength of 660 nm from an LED light as a light source and measuring the amount of 90-degree side scattered light at 0.1 seconds intervals. The maximum measurement time was 300 seconds (number of data: 3000, 0.1 seconds intervals). The measurement of coagulation reaction was performed twice for each of three standard samples and ten concentration series samples.

1.3) Reaction P(i) and production of reaction velocity V(i)

Reaction P(i) was produced by subjecting the photometric data from each sample to smoothing processing including noise removal and then performing zero point adjustment treatment such that the scattered light intensity at the point of time of starting photometry is 0. The primary differential value V(i) was calculated from the P(i).

1.4) Calculation of fibrinogen concentration by percentage method

Since the reaction curve of low-fibrinogen samples continues to rise slowly until the end of measurement in some cases, it is not appropriate to define the maximum value Pmax of a reaction curve as the calculation start point in a percentage method. Accordingly, in the present Example, the calculation start point of a percentage method was determined based on the integrated value ratio Z(i) of reaction P(i) (see Patent Literature 1). The integrated value ratio Z(i) reflects the trend of change in P(i). That is, Z(i) is large in the reaction initial stage in which P(i) increases and then asymptotically approaches 1 as P(i) approaches the plateau. In the present Example, Z(i) was calculated from P(i) by the following equation, and the point at which Z(i) became smaller than a threshold Zs was defined as the calculation start point of a percentage method. Zs was set to 1.05.

$$Z(i) = \{P(i + 1) + P(i + 2) + ... + P(i + m)\}/\{P(i - m) + P(i - m + 1) + ... + P(i - 1)\} \quad (m = 20)$$

Coagulation times of three standard samples and ten concentration series samples were measured by a percentage method. That is, when P(i) at the calculation start point was defined as 100%, the point of time at which P(i) arrived at 63% was determined as the coagulation time. The coagulation time of each sample was measured twice based on the coagulation reaction measurement each performed twice. Regarding the coagulation times measured twice for each of the three standard samples, averages of the duplicate measurements were calculated, and logarithms of the averages were plotted with respect to the logarithms of [Fbg] (mg/dL) of the standard samples to produce a standard curve by a percentage method. According to the produced standard curve, the fibrinogen concentration ([Fbg] arithmetic value by a percentage method, mg/dL) of each of the concentration series samples was calculated.

1.5) Calculation of fibrinogen concentration based on maximum velocity ratio

Maximum reaction velocity Vmax was detected from the V(i) obtained for each sample by the same process as in Example 1. The velocity threshold Vs was determined as a function represented by the following equation according to the process of Reference Example 1 described later. Ks was set to 1.1.

$$Vs(i) = 19 + (9357/i^2) \text{ [i represents a measuring point]}.$$

Time Te giving V(i) = Vmax × S% and time Tc giving P(Tc) = P(Te) × 63% were determined (S = 5, 10, 15, 20, 25, 30, 35, 40, 45, 50, 55, 60, 65, 70, 75, 80, 85, 90, and 95), and $Tc_5$ to $Tc_{95}$ were obtained. $Tc_5$ to $Tc_{95}$ of ten concentration series samples were calculated twice for each to obtain data for 20 samples. In addition, $Tc_5$ to $Tc_{95}$ of the three standard samples were calculated twice for each, and averages of the duplicate measurements were calculated. Logarithms of the averages were plotted with respect to the logarithms of [Fbg] (mg/dL) of the standard samples to produce a standard curve by Tc. According to the produced standard curve, the fibrinogen concentration ([Fbg] arithmetic value by maximum velocity ratio, mg/dL) of each of the concentration series samples was calculated.

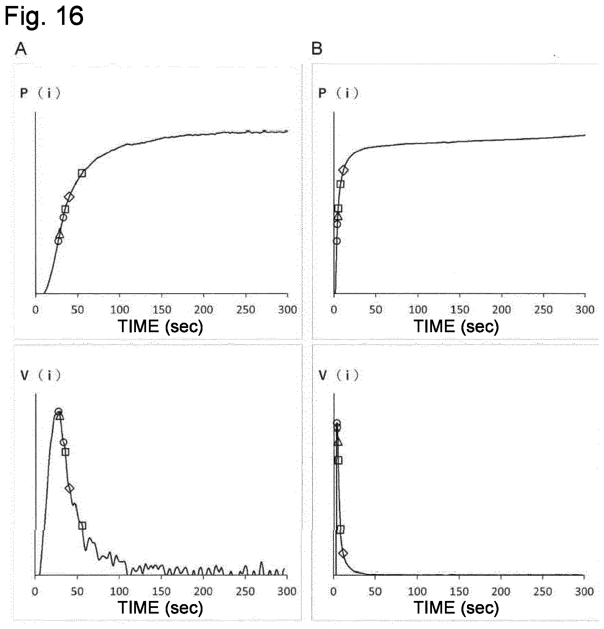

Figure 16 shows examples of measured Te and Tc and coagulation time by a percentage method. A and B of Figure 16 show the P(i) (the above) and V(i) (the below) of samples of which the [Fbg] was the minimum (98 mg/dL) and maximum (980 mg/dL), respectively, together with the calculation start point (rhombus) by an integrated value ratio, coagulation time (triangle) by a percentage method based on the calculation start point, $Te_{75}$ and $Te_{30}$ (square), and $Tc_{75}$ and $Tc_{30}$ (circle). In both A and B of Figure 16, $Tc_{75}$ and $Tc_{30}$ marked with circles were close to the coagulation time by a percentage method (conventional method) marked with a triangle, and it was suggested that they reflect coagulation time.

2. Evaluation of precision of fibrinogen concentration measurement based on Tc

2.1) Correlation analysis

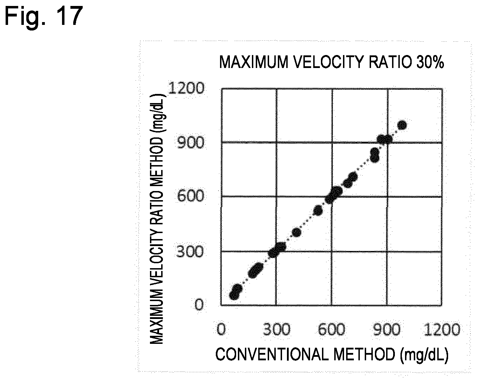

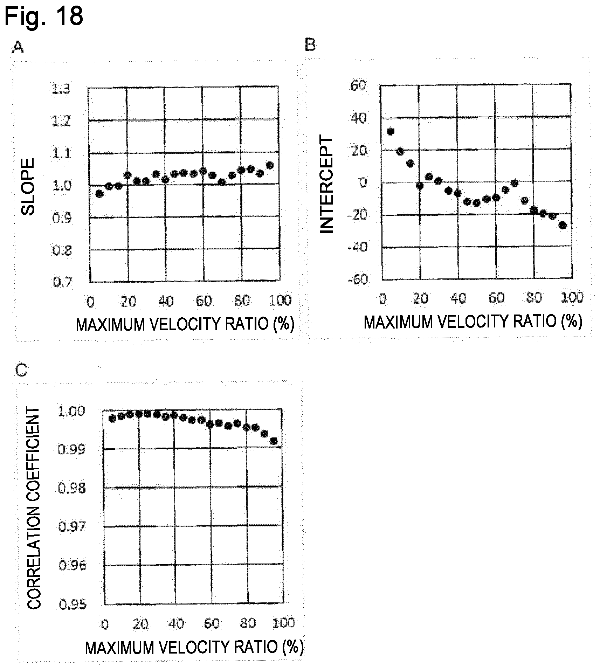

Figure 17 shows a primary regression line of [Fbg] arithmetic value (mg/dL) at $Tc_{30}$ with respect to [Fbg] arithmetic value by a percentage method, based on the data (n = 10 × 2) of concentration series samples. The [Fbg] arithmetic value based on $Tc_{30}$ had a high correlation with the [Fbg] arithmetic value by a percentage method. Figure 18 shows the slopes, intercepts, and correlation coefficients of [Fbg] arithmetic values calculated from $Tc_5$ to $Tc_{95}$ with respect to [Fbg] arithmetic values by a percentage method. In the range of $Tc_5$ to $Tc_{95}$, the slopes of the regression lines were 0.97 to 1.06, the intercepts were -27.1 to 32.0, and the correlation coefficients were 0.992 to 0.999 (A to C of Figure 18). It was confirmed that the fibrinogen concentration measuring method based on maximum velocity ratio leads to the results similar to those of a standard method based on a percentage method and accordingly has performance satisfying In-vitro diagnostic drug approval criteria, "comparing to control measurement method, the correlation coefficient is 0.9 or more, and the slope of regression line equation is 0.9 to 1.1", by the Ministry of Health, Labour and Welfare.

2.2) Accuracy of measurement

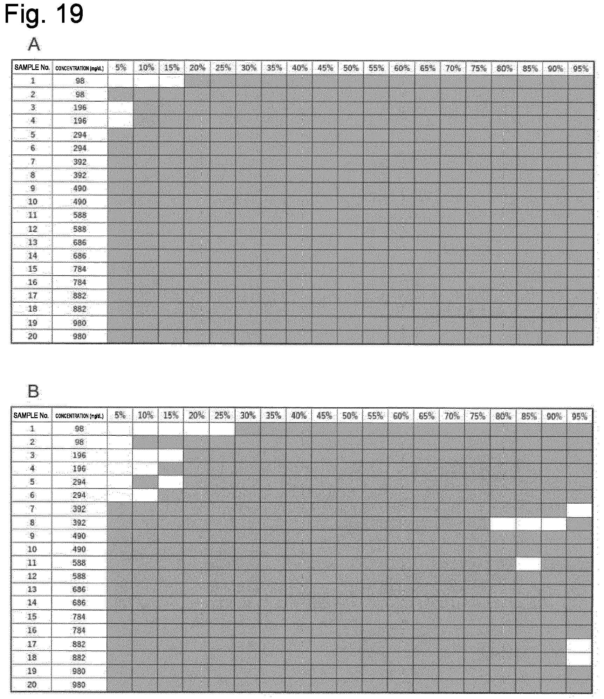

[Fbg] arithmetic values based on $Tc_5$ to $Tc_{95}$ regarding the data (10 samples × 2 of Table 4) of the concentration series samples were compared to expected values (Fbg concentrations of samples shown in Table 4). Each row of the tables of A and B of Figure 19 represents Tc (from the left, $Tc_5$ to $Tc_{95}$) of each sample. When the error between Tc and the expected value is within ±10% (A of Figure 19) or ±5% (B of Figure 19), it is represented by gray color. $Tc_{20}$ to $Tc_{95}$ of all samples agreed with the expected values within an error of ±10%, and $Tc_{30}$ to $Tc_{75}$ of all samples agreed with the expected values within an error of ±5%.

Example 4: Accuracy evaluation

The accuracy of a coagulation time measuring method based on the maximum velocity ratio by the present method was compared to the method described in Patent Literature 1 (a coagulation time measuring method using the integrated value ratio Z(i) of reaction P(i)). Z(i) is a ratio of the integrated value of P(i) in a first measurement interval and the integrated value of P(i) in a second measurement interval adjacent to the first interval and reflects the trend of change in P(i). Z(i) is large in the reaction initial stage in which P(i) increases and then asymptotically approaches 1 as P(i) approaches the plateau. In the method described in Patent Literature 1, the time at which Z(i) satisfies a prescribed value after Z(i) became smaller than a threshold Zs is determined as coagulation time. The coagulation time measuring method based on Z(i) is disclosed as a highly reliable coagulation time measuring method that can accurately measure coagulation time of not only a normal sample but also an abnormal sample, such as a low-fibrinogen sample. In the present Example, the accuracy of the coagulation time measuring method according to the present method based on maximum velocity ratio was evaluated by comparison with the coagulation time measuring method using integrated value ratio.

1. Method

1.1) Sample

Sample A: a sample prepared by adding unfractionated heparin to a normal plasma such that the unfractionated heparin concentration was 0.9 IU/mL (APTT: 139.0 seconds); and
Sample B: a sample having largest APTT (126.2 seconds) of heparin-containing plasm used in Example 1.

1.2) Coagulation reaction measurement

The same as Example 1.

1.3) Production of reaction P(i), reaction velocity V(i), and integrated value ratio Z(i)

Reaction P(i) was produced by subjecting the photometric data from each sample to smoothing processing including noise removal and then performing zero point adjustment treatment such that the scattered light intensity at the point of time of starting photometry is 0. The primary differential value V(i) was calculated from the P(i). The integrated value ratio Z(i) of the reaction P(i) was calculated by the following equation.

$$Z(i) = \{P(i + 1) + P(i + 2) + ... + P(i + m)\}/\{P(i -$$

$$m) + P(i - m + 1) + ... + P(i - 1)\} \quad (m = 20)$$

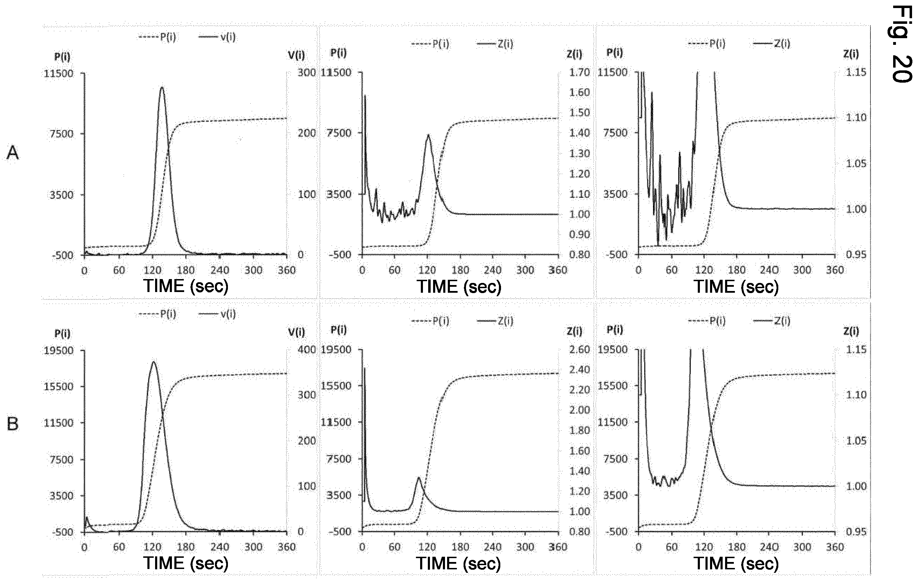

Figure 20 shows graphs of P(i) and V(i) (the left), P(i) and Z(i) (the middle), and enlarged Z(i) (the right) of two samples A (the above) and B (the below). In the sample A, immediately after the start of measurement, no clear peak (initial peak) was observed in V(i), but a plurality of initial peaks was observed in Z(i). It was inferred that these initial peaks reflect the turbulence in turbidity when a sample and a mixed solution of reagents are mixed in the reaction container (cuvette). In sample B, immediately after the start of measurement, in both V(i) and Z(i), initial peaks, which are inferred to reflect initial abnormal reaction (so-called early reaction), were observed. In both cases, the initial peak of V(i) was small compared to the initial peak of Z(i), and was quite small compared to the peak (reaction peak) that appears after the start of coagulation reaction (rising of P(i)). It was revealed from this that the reaction peak of V(i) can be easily distinguished from the initial peak. On the other hand, since the initial peak of Z(i) was larger than the reaction peak, it was revealed that in order to correctly recognize the reaction peak of Z(i), it is necessary to establish decision criteria for avoiding false detection of the initial peak. Accordingly, it was obvious that the present method based on maximum velocity ratio is resistant to measurement noises compared to the method using integrated value ratio and can more accurately measure the coagulation time.

Reference Example 1: Determination of Vs, Ks, and Ws

Processes for calculating velocity threshold Vs, maximum value continuation width threshold Ks, and peak width threshold Ws based on data of the reference sample pack will be described. As the reference sample packs, 24 samples used in Example 1 (hereinafter, APTT group), 23 samples used in Example 2 (hereinafter, PT group), or the data of 20 samples used in Example 3 (hereinafter, Fbg group) were used. In addition, control P-N for Coagpia (manufactured by Sekisui Medical Co., Ltd.), control P-N I and control P-N II, which are used as precision control samples were also included in the reference sample pack of each group.

1.1 Process of setting fixed value Vs

Regarding each sample of the reference sample pack, the maximum value Vmax of reaction velocity V(i) during the measurement period was determined. The smallest value of the Vmax obtained from each sample was determined, and 50% of the smallest Vmax was defined as Vs. The determined Vs was 36 in the APTT group, 107 in the PT group, and 17 in the Fbg group.

1.2 Process of setting function Vs

1) Control P-N I and control P-N II were used as "normal control" and "abnormal control", respectively. The normal control had the APTT and the Fbg concentration near the center within the reference range. In the abnormal control, the APTT extended over the reference range, and the Fbg concentration was a lower value than the reference range. The reference range is the range of 95% in the center of APTT or Fbg concentration distribution (distribution excluding 2.5% on both sides) of an existing healthy subject pack.
2) The coefficient of a function expression was determined using the measurement data of the normal control and the abnormal control. The maximum velocity time VmaxT of the normal control was defined as X1, and the maximum velocity Vmax was defined as Y1. In the abnormal control, similarly, VmaxT and Vmax were defined as X2 and Y2, respectively.
3) In order to calculate Vs that is smaller than Vmax in the reference sample pack, X1 and X2 were multiplied by a coefficient C (preferably $50\% \leq C \leq 90\%$) to obtain P1 and P2, respectively. In the present Reference Example, C was set to 60%.
4) The function expression was determined as $y = b + a/x^2$, and a and b were determined from simultaneous equations obtained by substituting P1 and Y1 for x and y of the expression and also substituting P2 and Y2 for x and y.
5) Vs was calculated from the determined a and b by an equation $Vs = b + a/i^2$. The calculation equations of Vs determined for the APTT group, PT group, and Fbg group were as follows:

APTT group: Vs(i) = -83 + (158214/i$^2$),
PT group: Vs(i) = -148 + (51935/i$^2$),
Fbg group: Vs(i) = 19 + (9357/i$^2$).

However, when the Vs determined by the above equation is less than the fixed value determined in the paragraph 1.1., the fixed value was defined as Vs.

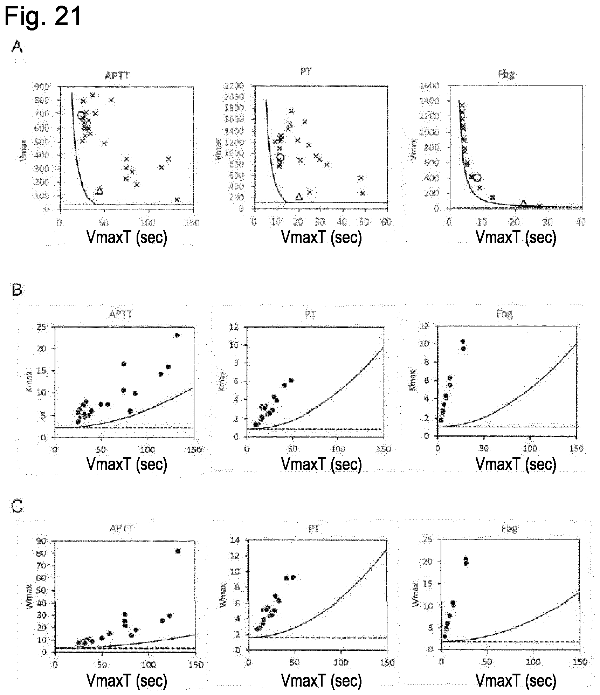

A of Figure 21 shows the distributions of Vmax and calculated Vs of, from the left, the APTT group, the PT group, and the Fbg group. Vmax was plotted with respect to VmaxT. In the graphs, the circle mark indicates data of a normal control, the triangle indicates data of an abnormal control. The dotted line indicates fixed value Vs, and the solid line indicates function Vs. In the graphs, the function Vs is represented as a function of time t (sec). In every group, the Vmax tends to decrease with an increase of VmaxT, and as a result, the function Vs is represented as a curve that decreases with time and is eventually substituted by the fixed value Vs.

2.1 Process of setting Ks

The maximum value (Kmax) of K(i) changes depending on the detection end point Tt, i.e., the value of V(Tt)/VmaxO ratio. K(i) recedes from VmaxT with an increase in Tt (a decrease in V(Tt)/VmaxO ratio), and therefore Kmax increases. K(i) approaches VmaxT with a decrease in Tt (an increase in V(Tt)/VmaxO ratio), and therefore Kmax decreases. The distribution of Kmax was investigated using the measurement data of the reference sample pack and varying the setting of the V(Tt)/VmaxO ratio (%) from 50% to 90% in 10% increments. As shown in Table 5, it was confirmed that the minimum and maximum values in the distribution of Kmax are affected by the V(Tt)/VmaxO ratio to show the trend as described above.

[Table 5]

| APTT group Kmax | | | | | |
|---|---|---|---|---|---|
| V(Tt)/Vmax0 ratio (%) | 50% | 60% | 70% | 80% | 90% |
| Kmax minimum value | 5.3 | 4.5 | 3.7 | 2.9 | 2.0 |
| Kmax maximum value | 35.4 | 31.7 | 23.1 | 12.8 | 8.6 |
| PT group Kmax | | | | | |
| V(Tt)/Vmax0 ratio (%) | 50% | 60% | 70% | 80% | 90% |
| Kmax minimum value | 2.1 | 1.8 | 1.4 | 1.1 | 0.7 |
| Kmax maximum value | 9.4 | 7.6 | 6.1 | 4.5 | 3.0 |
| Fbg group Kmax | | | | | |
| V(Tt)/Vmax0 ratio (%) | 50% | 60% | 70% | 80% | 90% |
| Kmax minimum value | 2.8 | 2.2 | 1.8 | 1.4 | 0.9 |
| Kmax maximum value | 14.8 | 11.9 | 10.3 | 8.4 | 4.0 |

B of Figure 21 shows the distributions of Kmax and calculated Ks when the V(Tt)/VmaxO ratio was set to 70% in, from the left, the APTT group, the PT group, and the Fbg group. Kmax was plotted with respect to VmaxT. It was revealed from these results that Kmax depends on the time of the reaction velocity peak, consequently the coagulation time, that is, Kmax increases with an increase in coagulation time, and Kmax decreases with a decrease in coagulation time. Thus, it was revealed that since Kmax tends to depend on time, Ks may be defined as a function of time or measuring point. A calculation equation: Ks(t) = constant b + coefficient a $\times$ t$^2$ (solid line in each graph) could be determined from the data in the graphs. The constant b was defined as a fixed value of Ks (dotted line in each graph) and was set as a value of 60% of the smallest Kmax, and was 2.2 seconds in the APTT group, 0.8 seconds in the PT group, and 1.1 seconds in the Fbg group. The coefficient a was appropriately set not to overlap the distribution of Kmax and was 0.0004 in every group.

3.1 Process of setting Ws

The maximum value (Wmax) of W(i) had the same tendency as Kmax, i.e., a tendency of depending on time. As shown in Table 6, the minimum and maximum values in the distribution of Wmax were investigated by varying the set value of V(Tt)/VmaxO ratio (%) from 50% to 90% in 10% increments.

[Table 6]

| APTT group Wmax | | | | | |
|---|---|---|---|---|---|
| V(Tt)/Vmax0 ratio (%) | 50% | 60% | 70% | 80% | 90% |
| Wmax minimum value | 7.9 | 6.8 | 5.7 | 4.5 | 3.2 |
| Wmax maximum value | 102.7 | 91.8 | 81.9 | 63.6 | 29.9 |
| PT group Wmax | | | | | |
| V(Tt)/Vmax0 ratio (%) | 50% | 60% | 70% | 80% | 90% |
| Wmax minimum value | 3.9 | 3.4 | 2.7 | 2.1 | 1.4 |
| Wmax maximum value | 13.6 | 11.3 | 9.3 | 7.5 | 5.4 |
| Fbg group Wmax | | | | | |
| V(Tt)/Vmax0 ratio (%) | 50% | 60% | 70% | 80% | 90% |
| Wmax minimum value | 4.1 | 3.5 | 3.1 | 2.7 | 2.0 |
| Wmax maximum value | 27.8 | 23.6 | 20.7 | 17.3 | 10.6 |

C of Figure 21 shows the distributions of Wmax and calculated Ws when the V(Tt)/VmaxO ratio was set to 70% in, from the left, the APTT group, the PT group, and the Fbg group. Wmax was plotted with respect to VmaxT. It was revealed that Wmax increases with an increase in VmaxT, and Wmax decreases with a decrease in VmaxT. A calculation equation: Ws(t) = constant b + coefficient a $\times$ t$^2$ (solid line in each graph) could be determined from the data in the graphs. The constant b was defined as a fixed value of Ws and was set as a value of 60% of the smallest Wmax, and was 3.4 seconds in the APTT group, 1.6 seconds in the PT group, and 1.9 seconds in the Fbg group. The coefficient a was appropriately set not to overlap the distribution of Wmax and was 0.0005 in every group.

Reference Example 2

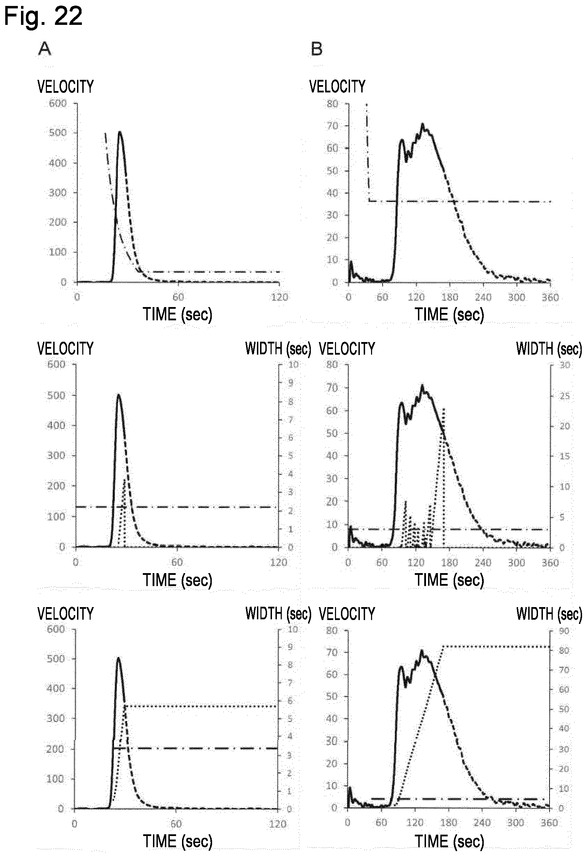

Figure 22 shows relationships between reaction velocity V(t) and velocity threshold Vs (the above), maximum value continuation width K(t) and Ks (the middle), and peak width W(t) and Ws (the below) in the sample (A) with the smallest APTT and the sample (B) with the largest APTT in Example 1 during the Vmax detection process. In Figure 22, V(t) is indicated by a solid line until the point of time at which V(t), after Vmax, arrived at 70% of Vmax (detection end point Tt) and thereafter by a dotted line. W(t) indicates 70% peak width. Vs was calculated by the following equation according to the above-described Reference Example 1. However, when a calculated value was less than 36, Vs was set to 36. As Ks and Ws, the following fixed values were used.

$$\mathtt{Vs(t) \ = \ -83 \ + \ 158214/t^2 \ [t \ is \ time \ (sec)]}$$

Ks = 2.2
Ws = 3.4

As shown in Figure 22, Vs (alternate long and short dash) following the above equation was very large in the reaction initial stage but decreased with time. It was revealed that false detection of the initial peak derived from early reaction or noises in the reaction initial stage can be prevented by setting Vs to vary as above. In addition, as shown in Figure 22, K(t) indicated by a thin dotted line shows one sharp triangular shape exceeding the threshold Ks in A of the figure, but in B of the figure, K(t) fluctuates up and down corresponding to peak appearance of V(t) and exceeded the threshold Ks (alternate long and short dash). As shown in the below of Figure 22, W(t) indicated by a thin dotted line increased with time in both A and B of the figure, exceeded the threshold Ws (alternate long and short dash), and became maximum value Wmax when V(t) exceeded VmaxT and arrived at 70% of Vmax. Wmax was 5.7 seconds in A of the Figure due to its sharp peak shape and was 82 seconds in B of the Figure due to the bimodal wide peak shape. In both A and B, K(t) exceeded Ks at the detection end point Tt, and W(t) exceeded Ws. It was revealed from these results that Vmax can be correctly detected by using K(t) or W(t) not only when V(t) has a single peak arriving at Vmax as in A, but also when a pre-peak appears before the reaction velocity V arrives at Vmax as in B.

**Claims**

1.  A method for measuring blood coagulation time, comprising:

    acquiring coagulation reaction P(i) of a subject specimen and reaction velocity V(i) as a differential value of the reaction P(i), where i represents a measuring point;
    calculating a calculation start point Te by using a ratio of V(i) to maximum reaction velocity as an index; and
    calculating time Tc giving $P(Tc) = P(Te) \times N\%$ (0 < N < 100) and determining the Tc as blood coagulation time.

2.  The method according to Claim 1, wherein the maximum reaction velocity is a maximum value of V(i) acquired so far.

3.  The method according to Claim 1 or 2, wherein the maximum reaction velocity is a maximum value of V(i) acquired after V(i) exceeds a threshold Vs until a latest measuring point.

4.  The method according to any one of Claims 1 to 3, wherein time at which V(i) reaches S% (S is a prescribed value within a range of from 5 to 95) of a maximum reaction velocity after V(i) arrives at the maximum reaction velocity is calculated as the calculation start point Te.

5.  The method according to Claim 4, wherein

    time at which V(i) reaches S% (S is a prescribed value within a range of from 5 to 95) of a maximum reaction velocity in a case where a maximum value continuation width K(i) exceeds a threshold Ks after V(i) arrived at the maximum reaction velocity is calculated as the calculation start point Te, where
    the maximum value continuation width K(i) is the time or the number of measuring points at which V(i) at i continues to be a certain value after arriving at the maximum reaction velocity.

6.  The method according to Claim 4 or 5, wherein

    time at which V(i) is S% (S is a prescribed value within a range of from 5 to 95) of the maximum reaction velocity in a case where a peak width W(i) exceeds a threshold Ws is calculated as the calculation start point Te, where
    the peak width W(i) is the time width or the number of measuring points between a latest i and a point where V(i) is X% of a maximum reaction velocity and before it reaches the maximum reaction velocity, where
    X is 1 or more and less than 100.

7.  A method for measuring coagulation factor concentration, comprising measuring coagulation factor concentration of a subject specimen based on blood coagulation time of the subject specimen measured by the method according to any one of Claims 1 to 6.

8.  The method according to Claim 7, wherein the coagulation factor is fibrinogen.

Fig. 1

Fig. 2

START OF MEASUREMENT

ACQUISITION OF
REACTION P(i) ——— STEP 1

ACQUISITION OF REACTION
VELOCITY V(i) ——— STEP 2

DETERMINATION OF MAXIMUM
REACTION VELOCITY Vmax ——— STEP 3

CALCULATION OF TIME (Te)
GIVING V(i) = Vmax × S% ——— STEP 4

CALCULATION OF TIME (Tc)
GIVING P(Tc) = P(Te) × N% ——— STEP 5

(COAGULATION TIME DETERMINATION)

Fig. 3

Fig. 4

Fig. 5

Fig. 6

# Fig. 7

Fig. 8

# Fig. 9

A

| SAMPLE No. | CONTROL [sec] | 5% | 10% | 15% | 20% | 25% | 30% | 35% | 40% | 45% | 50% | 55% | 60% | 65% | 70% | 75% | 80% | 85% | 90% | 95% |
|---|---|---|---|---|---|---|---|---|---|---|---|---|---|---|---|---|---|---|---|---|
| 1 | 27.2 | | | | | | | | | | | | | | | | | | | |
| 2 | 28.6 | | | | | | | | | | | | | | | | | | | |
| 3 | 29.9 | | | | | | | | | | | | | | | | | | | |
| 4 | 30.9 | | | | | | | | | | | | | | | | | | | |
| 5 | 31.2 | | | | | | | | | | | | | | | | | | | |
| 6 | 31.3 | | | | | | | | | | | | | | | | | | | |
| 7 | 31.7 | | | | | | | | | | | | | | | | | | | |
| 8 | 32.1 | | | | | | | | | | | | | | | | | | | |
| 9 | 34.2 | | | | | | | | | | | | | | | | | | | |
| 10 | 35.1 | | | | | | | | | | | | | | | | | | | |
| 11 | 35.4 | | | | | | | | | | | | | | | | | | | |
| 12 | 38.2 | | | | | | | | | | | | | | | | | | | |
| 13 | 38.3 | | | | | | | | | | | | | | | | | | | |
| 14 | 42.3 | | | | | | | | | | | | | | | | | | | |
| 15 | 55.0 | | | | | | | | | | | | | | | | | | | |
| 16 | 60.5 | | | | | | | | | | | | | | | | | | | |
| 17 | 75.1 | | | | | | | | | | | | | | | | | | | |
| 18 | 77.9 | | | | | | | | | | | | | | | | | | | |
| 19 | 78.4 | | | | | | | | | | | | | | | | | | | |
| 20 | 81.0 | | | | | | | | | | | | | | | | | | | |
| 21 | 87.6 | | | | | | | | | | | | | | | | | | | |
| 22 | 119.1 | | | | | | | | | | | | | | | | | | | |
| 23 | 126.2 | | | | | | | | | | | | | | | | | | | |
| 24 | 141.2 | | | | | | | | | | | | | | | | | | | |

B

| SAMPLE No. | CONTROL (sec) | 5% | 10% | 15% | 20% | 25% | 30% | 35% | 40% | 45% | 50% | 55% | 60% | 65% | 70% | 75% | 80% | 85% | 90% | 95% |
|---|---|---|---|---|---|---|---|---|---|---|---|---|---|---|---|---|---|---|---|---|
| 1 | 27.2 | | | | | | | | | | | | | | | | | | | |
| 2 | 28.6 | | | | | | | | | | | | | | | | | | | |
| 3 | 29.9 | | | | | | | | | | | | | | | | | | | |
| 4 | 30.9 | | | | | | | | | | | | | | | | | | | |
| 5 | 31.2 | | | | | | | | | | | | | | | | | | | |
| 6 | 31.3 | | | | | | | | | | | | | | | | | | | |
| 7 | 31.7 | | | | | | | | | | | | | | | | | | | |
| 8 | 32.1 | | | | | | | | | | | | | | | | | | | |
| 9 | 34.2 | | | | | | | | | | | | | | | | | | | |
| 10 | 35.1 | | | | | | | | | | | | | | | | | | | |
| 11 | 35.4 | | | | | | | | | | | | | | | | | | | |
| 12 | 38.2 | | | | | | | | | | | | | | | | | | | |
| 13 | 38.3 | | | | | | | | | | | | | | | | | | | |
| 14 | 42.3 | | | | | | | | | | | | | | | | | | | |
| 15 | 55.0 | | | | | | | | | | | | | | | | | | | |
| 16 | 60.5 | | | | | | | | | | | | | | | | | | | |
| 17 | 75.1 | | | | | | | | | | | | | | | | | | | |
| 18 | 77.9 | | | | | | | | | | | | | | | | | | | |
| 19 | 78.4 | | | | | | | | | | | | | | | | | | | |
| 20 | 81.0 | | | | | | | | | | | | | | | | | | | |
| 21 | 87.6 | | | | | | | | | | | | | | | | | | | |
| 22 | 119.1 | | | | | | | | | | | | | | | | | | | |
| 23 | 126.2 | | | | | | | | | | | | | | | | | | | |
| 24 | 141.2 | | | | | | | | | | | | | | | | | | | |

Fig. 10

Fig. 11

| SAMPLE No. | CONTROL (sec) | 0.5% | 1.0% | 1.5% | 2.0% | 2.5% | 3.0% | 3.5% | 4.0% | 4.5% | 5.0% |
|---|---|---|---|---|---|---|---|---|---|---|---|
| 1 | 27.2 | | | | | | | | | | |
| 2 | 28.6 | | | | | | | | | | |
| 3 | 29.9 | | | | | | | | | | |
| 4 | 30.9 | | | | | | | | | | |
| 5 | 31.2 | | | | | | | | | | |
| 6 | 31.3 | | | | | | | | | | |
| 7 | 31.7 | | | | | | | | | | |
| 8 | 32.1 | | | | | | | | | | |
| 9 | 34.2 | | | | | | | | | | |
| 10 | 35.1 | | | | | | | | | | |
| 11 | 35.4 | | | | | | | | | | |
| 12 | 38.2 | | | | | | | | | | |
| 13 | 38.3 | | | | | | | | | | |
| 14 | 42.3 | | | | | | | | | | |
| 15 | 55.0 | | | | | | | | | | |
| 16 | 60.5 | | | | | | | | | | |
| 17 | 75.1 | | | | | | | | | | |
| 18 | 77.9 | | | | | | | | | | |
| 19 | 78.4 | | | | | | | | | | |
| 20 | 81.0 | | | | | | | | | | |
| 21 | 87.6 | | | | | | | | | | |
| 22 | 119.1 | | | | | | | | | | |
| 23 | 126.2 | | | | | | | | | | |
| 24 | 141.2 | | | | | | | | | | |

Fig. 12

# Fig. 13

MAXIMUM VELOCITY RATIO 5%

Fig. 14

# Fig. 15

A

| SAMPLE No. | CONTROL (sec) | 5% | 10% | 15% | 20% | 25% | 30% | 35% | 40% | 45% | 50% | 55% | 60% | 65% | 70% | 75% | 80% | 85% | 90% | 95% |
|---|---|---|---|---|---|---|---|---|---|---|---|---|---|---|---|---|---|---|---|---|
| 1 | 9.5 | | | | | | | | | | | | | | | | | | | |
| 2 | 11.4 | | | | | | | | | | | | | | | | | | | |
| 3 | 11.4 | | | | | | | | | | | | | | | | | | | |
| 4 | 11.4 | | | | | | | | | | | | | | | | | | | |
| 5 | 11.5 | | | | | | | | | | | | | | | | | | | |
| 6 | 11.6 | | | | | | | | | | | | | | | | | | | |
| 7 | 11.7 | | | | | | | | | | | | | | | | | | | |
| 8 | 11.8 | | | | | | | | | | | | | | | | | | | |
| 9 | 12.1 | | | | | | | | | | | | | | | | | | | |
| 10 | 12.1 | | | | | | | | | | | | | | | | | | | |
| 11 | 15.8 | | | | | | | | | | | | | | | | | | | |
| 12 | 17.0 | | | | | | | | | | | | | | | | | | | |
| 13 | 17.2 | | | | | | | | | | | | | | | | | | | |
| 14 | 20.6 | | | | | | | | | | | | | | | | | | | |
| 15 | 21.9 | | | | | | | | | | | | | | | | | | | |
| 16 | 23.4 | | | | | | | | | | | | | | | | | | | |
| 17 | 25.2 | | | | | | | | | | | | | | | | | | | |
| 18 | 25.6 | | | | | | | | | | | | | | | | | | | |
| 19 | 28.6 | | | | | | | | | | | | | | | | | | | |
| 20 | 31.3 | | | | | | | | | | | | | | | | | | | |
| 21 | 34.8 | | | | | | | | | | | | | | | | | | | |
| 22 | 43.0 | | | | | | | | | | | | | | | | | | | |
| 23 | 51.6 | | | | | | | | | | | | | | | | | | | |

B

| SAMPLE No. | CONTROL (sec) | 5% | 10% | 15% | 20% | 25% | 30% | 35% | 40% | 45% | 50% | 55% | 60% | 65% | 70% | 75% | 80% | 85% | 90% | 95% |
|---|---|---|---|---|---|---|---|---|---|---|---|---|---|---|---|---|---|---|---|---|
| 1 | 9.5 | | | | | | | | | | | | | | | | | | | |
| 2 | 11.4 | | | | | | | | | | | | | | | | | | | |
| 3 | 11.4 | | | | | | | | | | | | | | | | | | | |
| 4 | 11.4 | | | | | | | | | | | | | | | | | | | |
| 5 | 11.5 | | | | | | | | | | | | | | | | | | | |
| 6 | 11.6 | | | | | | | | | | | | | | | | | | | |
| 7 | 11.7 | | | | | | | | | | | | | | | | | | | |
| 8 | 11.8 | | | | | | | | | | | | | | | | | | | |
| 9 | 12.1 | | | | | | | | | | | | | | | | | | | |
| 10 | 12.1 | | | | | | | | | | | | | | | | | | | |
| 11 | 15.8 | | | | | | | | | | | | | | | | | | | |
| 12 | 17.0 | | | | | | | | | | | | | | | | | | | |
| 13 | 17.2 | | | | | | | | | | | | | | | | | | | |
| 14 | 20.6 | | | | | | | | | | | | | | | | | | | |
| 15 | 21.9 | | | | | | | | | | | | | | | | | | | |
| 16 | 23.4 | | | | | | | | | | | | | | | | | | | |
| 17 | 25.2 | | | | | | | | | | | | | | | | | | | |
| 18 | 25.6 | | | | | | | | | | | | | | | | | | | |
| 19 | 28.6 | | | | | | | | | | | | | | | | | | | |
| 20 | 31.3 | | | | | | | | | | | | | | | | | | | |
| 21 | 34.8 | | | | | | | | | | | | | | | | | | | |
| 22 | 43.0 | | | | | | | | | | | | | | | | | | | |
| 23 | 51.6 | | | | | | | | | | | | | | | | | | | |

Fig. 16

Fig. 17

Fig. 18

## Fig. 19

A

B

## Fig. 20

## Fig. 21

Fig. 22

# EP 4 134 675 A1

## INTERNATIONAL SEARCH REPORT

International application No.

PCT/JP2021/014697

A. CLASSIFICATION OF SUBJECT MATTER
G01N 33/86(2006.01)i
FI: G01N33/86
According to International Patent Classification (IPC) or to both national classification and IPC

B. FIELDS SEARCHED

Minimum documentation searched (classification system followed by classification symbols)
G01N33/86

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 06-027115 A (INTERNATIONAL REAGENTS CORPORATION) 04 February 1994 (1994-02-04) entire text, all drawings, in particular, see claims, paragraphs [0006]-[0018], etc. | 1-8 |
| A | JP 06-249855 A (INTERNATIONAL REAGENTS CORPORATION) 09 September 1994 (1994-09-09) entire text, all drawings, in particular,see claims, paragraphs [0006]-[0017], etc. | 1-8 |
| A | WO 2014/162878 A1 (HITACHI HIGH-TECHNOLOGIES CORP.) 09 October 2014 (2014-10-09) entire text, all drawings, in particular, see claims, etc. | 1-8 |
| A | JP 2008-209350 A (MATSUSHITA ELECTRIC INDUSTRIAL CO., LTD.) 11 September 2008 (2008-09-11) entire text, all drawings, in particular, see claims, etc. | 1-8 |
| A | JP 2010-217059 A (SHIMADZU CORPORATION) 30 September 2010 (2010-09-30) entire text, all drawings, in particular, see claims, etc. | 1-8 |

☒ Further documents are listed in the continuation of Box C.　　☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 June 2021 (18.06.2021) | 29 June 2021 (29.06.2021) |

| Name and mailing address of the ISA/ Japan Patent Office 3-4-3, Kasumigaseki, Chiyoda-ku, Tokyo 100-8915, Japan | Authorized officer |
| | Telephone No. |

Form PCT/ISA/210 (second sheet) (January 2015)

43

**INTERNATIONAL SEARCH REPORT**

International application No.

PCT/JP2021/014697

C (Continuation).   DOCUMENTS CONSIDERED TO BE RELEVANT

| Category* | Citation of document, with indication, where appropriate, of the relevant passages | Relevant to claim No. |
|---|---|---|
| A | JP 04-318463 A (KDK CORP.) 10 November 1992 (1992-11-10) entire text, all drawings, in particular, see claims, etc. | 1-8 |
| A | JP 2003-169700 A (SYSMEX CORPORATION) 17 June 2003 (2003-06-17) entire text, all drawings, in particular, see claims, etc. | 1-8 |
| A | JP 2019-086517 A (SEKISUI MEDICAL CO., LTD.) 06 June 2019 (2019-06-06) entire text, all drawings, in particular, see claims, etc. | 1-8 |

Form PCT/ISA/210 (continuation of second sheet) (January 2015)

**INTERNATIONAL SEARCH REPORT**
Information on patent family members

| International application no. |
| --- |
| PCT/JP2021/014697 |

| Patent Documents referred in the Report | Publication Date | Patent Family | Publication Date |
| --- | --- | --- | --- |
| JP 06-027115 A | 04 Feb. 1994 | (Family: none) | |
| JP 06-249855 A | 09 Sep. 1994 | (Family: none) | |
| WO 2014/162878 A1 | 09 Oct. 2014 | US 2016/0291046 A1 entire text, all drawings, in particular, see claims, etc. EP 2982988 A1 | |
| JP 2008-209350 A | 11 Sep. 2008 | (Family: none) | |
| JP 2010-217059 A | 30 Sep. 2010 | (Family: none) | |
| JP 04-318463 A | 10 Nov. 1992 | (Family: none) | |
| JP 2003-169700 A | 17 Jun. 2003 | US 2003/0138962 A1 entire text, all drawings, in particular, see claims, etc. EP 1316802 A2 | |
| JP 2019-086517 A | 06 Jun. 2019 | (Family: none) | |

Form PCT/ISA/210 (patent family annex) (January 2015)

## REFERENCES CITED IN THE DESCRIPTION

**Patent documents cited in the description**

- JP 6249855 A